# EUROPEAN PATENT APPLICATION

(11) **EP 4 520 261 A1**
(43) Date of publication of application: **12.03.2025**
(21) Application number: 23918034.2
(22) Date of filing: 14.07.2023
(51) Int. Cl.: A61B 5/0245, A61B 5/28, A61B 5/308, A61B 5/00, A61B 5/282

(54) **ELECTROCARDIOGRAM MONITORING SYSTEM AND METHOD**

(71) Applicant: Shenzhen Shokz Co., Ltd., Shenzhen, Guangdong 518108 (CN)
(72) Inventor: Zhou, Xin, Shenzhen, Guangdong 518108 (CN); Li, Meiqi, Shenzhen, Guangdong 518108 (CN); Su, Lei, Shenzhen, Guangdong 518108 (CN); Zhang, Yuxiang, Shenzhen, Guangdong 518108 (CN); Ning, Yixuan, Shenzhen, Guangdong 518108 (CN); Su, Qi, Shenzhen, Guangdong 518108 (CN); Liu, Jia, Shenzhen, Guangdong 518108 (CN); Liao, Fengyun, Shenzhen, Guangdong 518108 (CN); Qi, Xin, Shenzhen, Guangdong 518108 (CN)
(74) Representative: Wang, Bo
(86) International application number: PCT/CN2023/107581
(87) International publication number: WO 2025/015473

(57) **Abstract**

An electrocardio monitoring system and method are provided. The electrocardio monitoring system includes at least two sets of electrocardio channels and a processing circuit. Each of the at least two sets of electrocardio channels includes at least two electrodes, and each of the at least two sets of electrocardiographic channels is configured to collect signals from a human body through the at least two electrodes, respectively. The processing circuit is configured to extract the electrocardio signal from the signals collected by the at least two sets of electrocardiographic channels.

## Description

### TECHNICAL FIELD

The present disclosure relates to the field of electrocardio monitoring, and in particular, to electrocardio monitoring systems and methods.

### BACKGROUND

Electrocardio monitoring devices currently used in the human body are continuously optimized. However, the acquisition of electrocardio signals is still strongly interfered with interference signals. The interference signals include human body electromyographic signals, resulting in that electrocardio signals cannot accurately reflect the cardiac activity of the human body.

Accordingly, it is desirable to provide an electrocardio monitoring system and method capable of substantially reducing interference signals.

### SUMMARY

One of the embodiments of the present disclosure provides an electrocardio monitoring system, including at least two sets of electrocardio channels and a processing device. Each of the at least two sets of electrocardio channels may include at least two electrodes. Each of the at least two sets of electrocardio channels may be configured to collect signals from a human body through the at least two electrodes, the signals including an electrocardio signal and a noise signal. The processing circuit may be configured to extract the electrocardio signal from the signals collected by the at least two sets of electrocardio channels.

One of the embodiments of the present disclosure further provides an electrocardio monitoring method. The method may include collecting signals from a human body through at least two sets of electrocardio channels. The signals may include an electrocardio signal and a noise signal, and each set of the at least two sets of electrocardio channels may include at least two electrodes. Each of the at least two sets of electrocardio channels may be configured to collect signals from the human body through the at least two electrodes. The method may also include extracting the electrocardio signal from the signals collected by the at least two sets of electrocardio channels.

Additional characteristics may be set forth in part in the following description and may become apparent to those skilled in the art by consulting the following and the accompanying drawings, or may be appreciated by the production or operation of examples. Characteristics of the present disclosure may be realized and obtained by practicing or using aspects of the methods, tools, and combinations outlined in the following detailed examples.

### BRIEF DESCRIPTION OF THE DRAWINGS

The present disclosure is further illustrated in terms of exemplary embodiments. These exemplary embodiments are described in detail with reference to according to the drawings. These embodiments are non-limiting exemplary embodiments, in which like reference numerals represent similar structures, and wherein:
FIG. 1 is a schematic diagram illustrating an application scenario of an electrocardio monitoring system according to some embodiments of the present disclosure;
FIG. 2 is a schematic diagram illustrating a structure of an electrocardio monitoring system according to some embodiments of the present disclosure;
FIG. 3A is a diagram illustrating modules of an electrocardio monitoring system according to some embodiments of the present disclosure;
FIG. 3B is a diagram illustrating modules of at least two sets of electrocardio channels according to some embodiments of the present disclosure;
FIG. 3C is a diagram illustrating modules of at least two sets of electrocardio channels according to some embodiments of the present disclosure;
FIG. 4 is a schematic diagram illustrating a whole human body according to some embodiments of the present disclosure;
FIG. 5A is a schematic diagram illustrating a structure of a wearable body according to some embodiments of the present disclosure;
FIG. 5B is a schematic diagram illustrating a structure of a wearable body according to some other embodiments of the present disclosure;
FIG. 5C is a schematic diagram illustrating a structure of a wearable body according to some other embodiments of the present disclosure;
FIG. 6 is a schematic diagram illustrating an ilium at the lower back of a human body according to some embodiments of the present disclosure;
FIG. 7 is a flowchart illustrating an exemplary process of measuring an electrocardio signal according to some embodiments of the present disclosure;
FIG. 8 is a schematic diagram illustrating a standardized waveform of an electrocardio signal according to some embodiments of the present disclosure;
FIG. 9 is a diagram illustrating a waveform of electrocardio channels and signals from a human body in a standing position collected by the electrocardio channels according to some embodiments of the present disclosure;
FIG. 10 is a diagram illustrating signals from a human body under an accelerated running state collected by different electrocardio channels according to some embodiments of the present disclosure;
FIG. 11 is a diagram illustrating signals from a human body under an accelerated running state collected by different electrocardio channels according to some embodiments of the present disclosure;
FIG. 12 is a diagram illustrating signals from a human body collected by different electrocardio channels according to some embodiments of the present disclosure; and
FIG. 13 is a flowchart illustrating an exemplary process of extracting an electrocardio signal according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. It should be understood that these exemplary embodiments are only to enable those of ordinary skill in the art to better understand and thus realize the present disclosure, and are not intended to limit the scope of the present disclosure in any way. Unless obviously obtained from the context or the context illustrates otherwise, the same numeral in the drawings refers to the same structure or operation.

As shown in the present disclosure and claims, unless the context clearly dictates otherwise, the words "a", "an", "one" and/or "the" are not intended to be specific in the singular and may include the plural. In general, the terms "comprise", "comprises", "comprising", "include", "includes", and/or "including", merely prompt to include operations and elements that have been clearly identified, and these operations and elements do not constitute an exclusive listing. The methods or devices may also include other operations or elements. The term "based on" is "based at least in part on." The term "one embodiment" means "at least one embodiment"; the term "another embodiment" means "at least one other embodiment".

In the description of the present disclosure, it is to be understood that the terms "front", "rear", "ear hook", "rear hook", etc. Indicate an orientation or positional relationship based only on that shown in the accompanying drawings, and are used only to facilitate the description of the present disclosure and simplify the description, and are not intended to indicate or imply that the device or element referred to must have a particular orientation, constructed and operated, and therefore are not to be construed as a limitation of the present disclosure.

Additionally, the terms "first" and "second" are used only for descriptive purposes and are not to be construed as indicating or implying relative importance or implicitly specifying the number of technical characteristics indicated. Therefore, the characteristic defined as "first", or "second" may expressly or impliedly include at least one such characteristic. In the description of the present disclosure, "a plurality of" means at least two, e.g., two, three, etc., unless explicitly and specifically limited otherwise.

In the present disclosure, unless otherwise expressly specified or limited, the terms "mounted", "connected", "connection", "fixed", etc. Are to be understood in a broad sense. For example, the connection may be a fixed connection, a removable connection, or a one-piece connection; a mechanical connection or an electrical connection; a direct connection, an indirect connection through an intermediate medium, a connection within two components, or an interactive relationship between two components, unless otherwise expressly limited. To one of ordinary skill in the art, the specific meaning of the above terms in the present disclosure may be understood on a case-by-case basis.

Embodiments of the present disclosure provide an electrocardio monitoring system and method. The electrocardio monitoring system includes a plurality of sets of different electrocardio channels and a processing circuit. Each of the plurality of sets of electrocardio channels includes at least two electrodes at a different distribution position, and each of the plurality of sets of electrocardio channels is configured to collect signals (e.g., an electrocardio signal and a noise signal) from a human body through the at least two electrodes. Then, the processing circuit extracts the electrocardio signal by processing a plurality of signals collected by the plurality of sets of different electrocardio channels. By setting the at least two electrodes corresponding to each set of electrocardio channels in different distribution positions, consistency of electrocardio signals collected by each set of electrocardio channels is better, and variability of noise signals is greater. Thus, in the subsequent processing, through signals of the human body collected by a plurality of sets of electrocardio channels, the electrocardio signal may be quickly separated from the noise signal, and thus the electrocardio signal may be extracted. Furthermore, the electrocardio signals may be recognized based on the signals of the human body collected by the plurality of sets of different electrocardio channels, thus improving the quality of the electrocardio signal to avoid the interference of the noise signal.

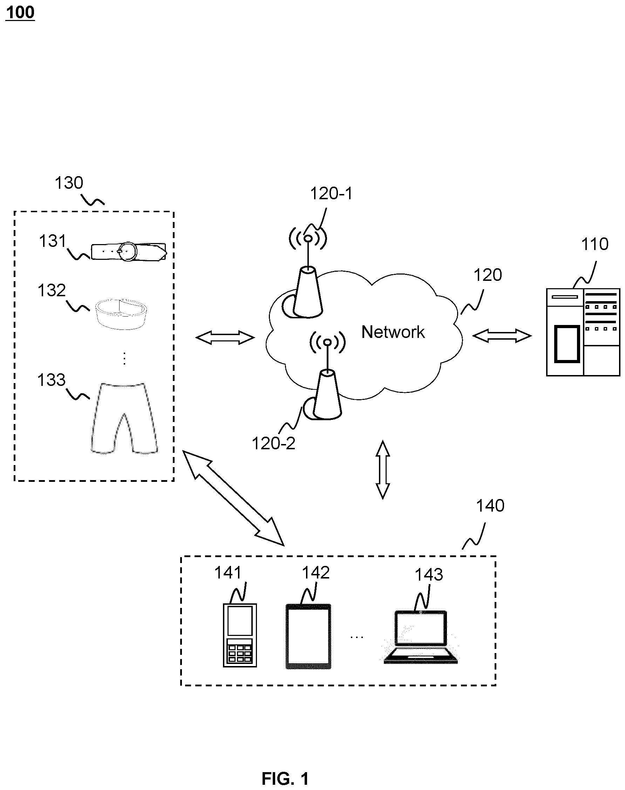

FIG. 1 is a schematic diagram illustrating an application scenario of an electrocardio monitoring system according to some embodiments of the present disclosure.

As shown in FIG. 1, the application scenario 100 may include a processing device 110, a network 120, a wearable body 130, and a terminal device 140. In some embodiments, the application scenario 100 may be applied to a plurality of scenarios such as physical exercise, psychological testing, clinical health monitoring, sleep monitoring, or the like.

In some embodiments, the processing device 110 may include a processing circuit. In some embodiments, the processing device 110 may be configured to process an electrical signal generated by the wearable body 130 and the terminal device 140. For example, the processing device 110 may receive signals (e.g., an electrocardio signal and a noise signal) from a human body transmitted by the wearable body 130 via the network 120 or a circuit. The processing device 110 may receive input information input by a user via the terminal device 140 through the network 120. In some embodiments, the processing device 110 may be configured to extract the electrocardio signal based on signals from the human body.

In some embodiments, the processing device 110 may be local or remote. For example, the processing device 110 may access information stored in the wearable body 130 and/or the terminal device 140 directly or via the network 120. In some embodiments, the processing device 110 may be directly connected to the wearable body 130 and/or the terminal device 140 to access information stored therein. For example, the processing device 110 may be disposed in the wearable body 130 and enable information interaction with the terminal device 140 via the network 120 or the circuit. When the circuit is used for information interaction, a carrier of the circuit may be a flexible circuit board or a printed circuit board. As another example, the processing device 110 may be located in the terminal device 140 and enable information interaction with the wearable body 130 via the network 120. In some embodiments, the processing device 110 may be executed on a cloud platform. For example, the cloud platform may include one of a private cloud, a public cloud, a hybrid cloud, an on-premises cloud, etc., or any combination thereof.

In some embodiments, the processing device 110 may include one or more sub-processing devices (e.g., a single-core processing device or a multi-core processing device). Merely by way of example, the processing device 110 may include a central processing unit (CPU), an application-specific integrated circuit (ASIC), an application-specific instruction processor (ASIP), a controller, a microcontroller unit, a microprocessor, etc., or any combination of the above.

The network 120 may facilitate the exchange of data and/or information between components in the application scenario 100. In some embodiments, one or more components of the application scenario 100 (e.g., the processing device 110, the wearable body 130, the terminal device 140) may send data and/or information via the network 120 to other components of the application scenario 100. For example, the electrical signal generated by the terminal device 140 may be transmitted to the processing device 110 via the network 120. In some embodiments, the network 120 may be any type of wired or wireless network. For example, the network 120 may include a wired network, a fiber optic network, a wide area network (WAN), a wireless area network (WLAN), a Bluetooth network, a near field communication (NFC) network, etc., or any combination thereof. In some embodiments, the network 120 may include one or more network entry and exit points. For example, the network 120 may include wired or wireless network entry and exit points, such as a base station and/or an inter-network switching point 120-1, an inter-network switching point 120-2, or the like, through which one or more of the components of the application scenario 100 may be connected to the network 120 to exchange data and/or information.

The wearable body 130 refers to a garment or device with wearable functionality. In some embodiments, the wearable body 130 may include but is not limited to, a belt 131, wrist guards 132, pants 133, or the like. The wearable body 130 may be configured to collect the signals from the human body (e.g., the electrocardio signal and the noise signal) and transmit the signal to the processing device 110 via the network 120. In some embodiments, the wearable body 130 may be provided with at least two sets of electrocardio channels, each of the at least two sets of electrocardio channels include at least two electrodes, and the at least two electrodes are configured to fit against the skin of the human body to collect the signals from the human body. It should be noted that the wearable body 130 is not limited to the belt 131, the wrist guards 132, and the pants 133 as shown in FIG. 1, but may also include other devices such as a skirt, a watch, an elbow protector, a shoulder protector, knee pads, socks, etc., which are not limited by the present disclosure, and any device that may be used with the present disclosure is within the scope of protection of the present disclosure.

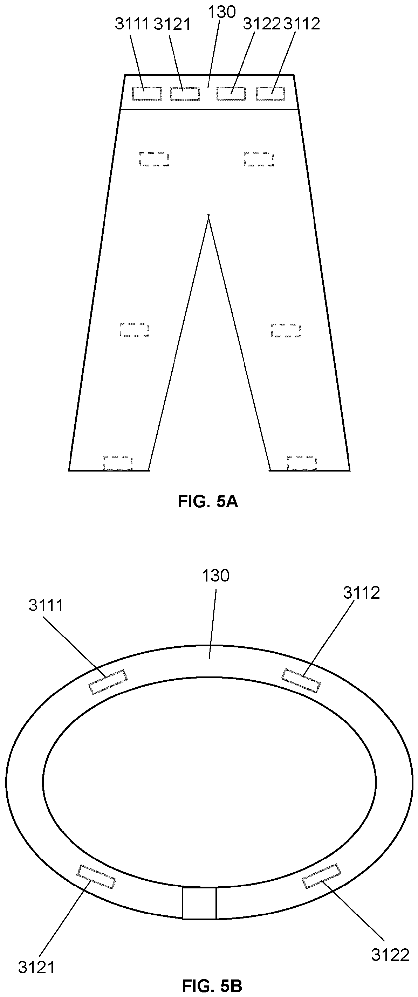

More descriptions regarding the wearable body may be found in FIGs.5A-5C and related descriptions thereof.

The terminal device 140 may be a device that interacts with the user. The terminal device 140 may receive the input information input by the user and send the input information over the network 120 to the processing device 110. In some embodiments, the terminal device 140 may receive feedback information sent by the processing device 110 via the network 120 and present the feedback information to the user. In some embodiments, the terminal device 140 may be a mobile smart terminal 141, a tablet 142, a laptop 143, or the like. In some embodiments, the terminal device 140 may be integrated on the processing device 110 and the wearable body 130. In some embodiments, the terminal device 140 may be other devices. For example, a cell phone, a smart home device, a smart mobile device, a smart wearable device (e.g., a virtual reality device, an augmented reality device, a smart watch, a smart bracelet), or any combination thereof.

In some embodiments, the application scenario 100 may also include other devices or components, such as a database. The database may be configured to store data. For example, the signals from the human body (e.g., the electrocardio signal and the electromyographic signal, etc.) received by the terminal device 140. In some embodiments, the database may store information obtained from the wearable body 130 and/or a mobile terminal device. In some embodiments, the database may include removable memory, volatile read-write memory (e.g., random access memory (RAM)), read-only memory (ROM), etc., or any combination thereof. In some embodiments, the database may be connected to the network 120 to communicate with one or more components of the application scenario 100 (e.g., the processing device 110, the wearable body 130, the terminal device 140, etc.). One or more of the components of the application scenario 100 may access data stored in the database via the network 120. In some embodiments, the database may be part of the processing device 110.

It should be noted that the above description of the electrocardio monitoring system and its modules is for descriptive convenience only, and does not limit the present disclosure to the scope of the cited embodiments. It should be understood that for a person skilled in the art, after understanding the principle of the system, it may be possible to arbitrarily combine each of the modules or form a sub-system to be connected to the other modules without departing from this principle. In some embodiments, the processing device 110, the network 120, the wearable body 130, and the terminal device 140 disclosed in FIG. 1 may be different modules in a system, or may be one module that implements the functions of two or more of the modules described above. For example, each module may share a common database, and each module may have its database. Such morphisms are within the scope of protection of the present disclosure.

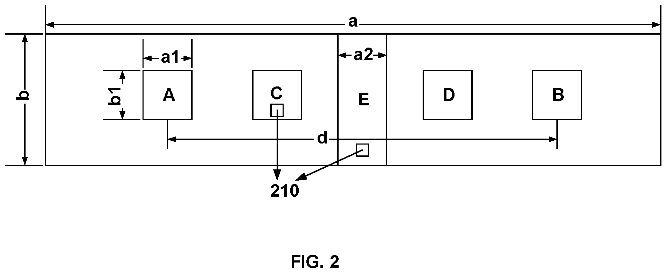

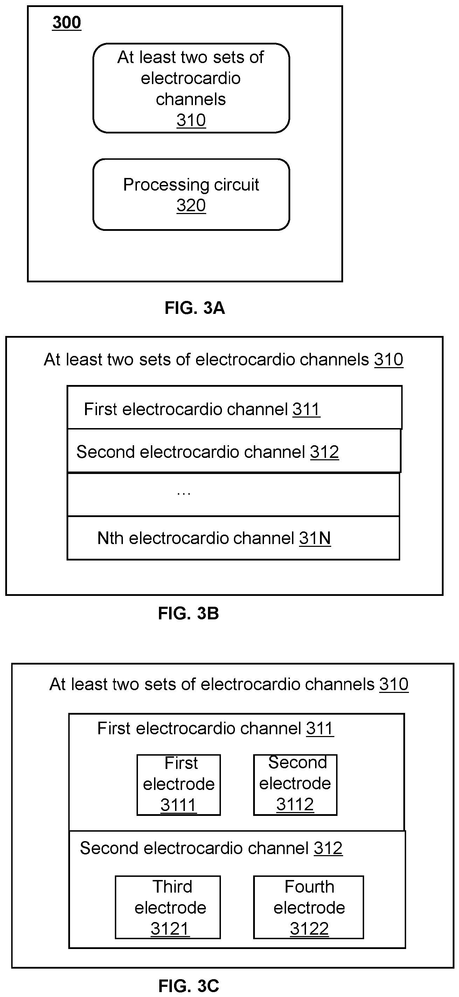

FIG. 2 is a schematic diagram illustrating a structure of an electrocardio monitoring system according to some embodiments of the present disclosure. FIG. 3A is a diagram illustrating modules of an electrocardio monitoring system according to some embodiments of the present disclosure. FIG. 3B is a diagram illustrating modules of at least two sets of electrocardio channels according to some embodiments of the present disclosure. FIG. 3C is a diagram illustrating modules of at least two sets of electrocardio channels according to some embodiments of the present disclosure.

As shown in FIG.2-FIG.3 A, an electrocardio monitoring system 300 may include at least two sets of electrocardio channels 310 and a processing circuit 320.

An electrocardio channel refers to an acquisition channel constituted by components configured to collect the electrocardio signal. In some embodiments, each of the at least two sets of electrocardio channels may include at least two electrodes (e.g., a first electrode, a second electrode), and the at least two sets of electrocardio channels 310 collect the signals from the human body through the at least two electrodes, respectively. Specific descriptions regarding the first electrode and the second electrode may be found in related descriptions hereinafter. In some embodiments, at least two electrodes corresponding to each of the at least two electrocardio channels may be configured to fit against the skin of the human body to collect the signals from the human body. In some embodiments, a count of electrodes may be two, three, etc. As shown in FIG. 2, electrodes of an electrocardio channel AB may include an electrode A, an electrode B (and may also include electrode E), etc.

In some embodiments, there may be a plurality of sets of electrocardio channels, such as 2 sets, 3 sets, etc. As shown in FIG. 3B, the at least two sets of electrocardio channels 310 may include a first electrocardio channel 311, a second electrocardio channel 312, and a Nth electrocardio channel 31N.

In some embodiments, a distribution position of each set of the at least two sets of electrocardio channels varies because a distribution position of the at least two electrodes corresponding to each set of the at least two sets of electrocardio channels varies. As shown in FIG. 2, based on positions of the electrode A and the electrode B corresponding to the electrocardio channel AB, and based on positions of electrode C and electrode D corresponding to an electrocardio channel CD, distribution positions of the electrocardio channel AB and the electrocardio channel CD are significantly different.

In some embodiments, each of the at least two sets of electrocardio channels may include two electrocardio electrodes (e.g., as shown in FIG. 3C, the first electrocardio channel 311 may include a first electrode 3111 and a second electrode 3112, and the second electrocardio channel 312 may include a third electrode 3121 and a fourth electrode 3122), and the two electrocardio electrodes may be affixed to different parts of the human body. Taking the first electrode and the second electrode as an example, a part to which the first electrode is affixed may have a first potential, and the part to which the second electrode is affixed may have a second potential. A difference between the first potential and the second potential may be used to reflect the signals from the human body, such as the cardiac signal and the noise signal. More descriptions regarding the noise signal may be found in related descriptions hereinafter).

In some embodiments, each of the at least two sets of electrocardio channels may also include a reference electrode. As shown in FIG. 2, the electrocardio channel AB may include the electrode E in addition to the electrode A and the electrode B. Understandably, the electrode E is the reference electrode.

The reference electrode may be configured to provide reference potentials for the two electrocardio electrodes (e.g., the first electrode and the second electrode) for subsequent processing of the first potential corresponding to the first electrode and the second potential corresponding to the second electrode using the processing circuit 320. In some embodiments, the reference electrode may be located between the first electrode and the second electrode, such that a potential difference value between the first potential corresponding to the first electrode and the reference electrode and a potential difference value between the second potential corresponding to the second electrode and the reference electrode are substantially equal, to facilitate subsequent processing of the processing circuit 320.

It should be noted that there is no limit to a count of reference electrodes, which may be one or more. For example, the reference electrodes may include a first reference electrode and a second reference electrode. The first reference electrode may be connected with the second reference electrode based on a wire, to make the two reference electrodes be of the same potential. At this time, the first reference electrode and the second reference electrode may be used as one reference electrode, so that a freedom degree of the wearable body 130 regarding a symmetry setting may be improved. Further, the plurality of electrocardio channels may share a single reference electrode or may each include at least one reference electrode.

In some embodiments, the noise signal may include the electromyographic signal and a motion artifact. The electromyographic signal refers to differential signals generated at the at least two electrodes based on a difference in distribution positions of at least two electrodes corresponding to each of the at least two sets of electrocardio channels and differences in muscle types and a force generation degree of muscles at the distribution position when the human body is exercising. The motion artifact refers to an interference signal formed due to the motion of the human body, which results in a change in contact impedance between the skin and the at least two electrodes corresponding to each of the at least two sets of electrocardio channels. Understandably, when the human body is in motion, the distribution positions of the at least two electrodes corresponding to each of the at least two sets of electrocardio channels are different. On the one hand, there is a variability in the contact impedance between the skin and the at least two electrodes at the different distribution positions. On the other hand, there is variability in a charge distribution of the contact interface between the skin and the at least two electrodes at different distribution positions and variability in the change in the charge distribution due to different pressure displacements. Thus, the plurality of motion artifacts collected based on the at least two electrodes corresponding to the plurality of sets of different electrocardio channels may also be different.

In some embodiments, the electrocardio signal and the noise signal (e.g., the electromyographic signal and the motion artifact, etc.) may be collected simultaneously based on the at least two sets of electrocardio channels 310.

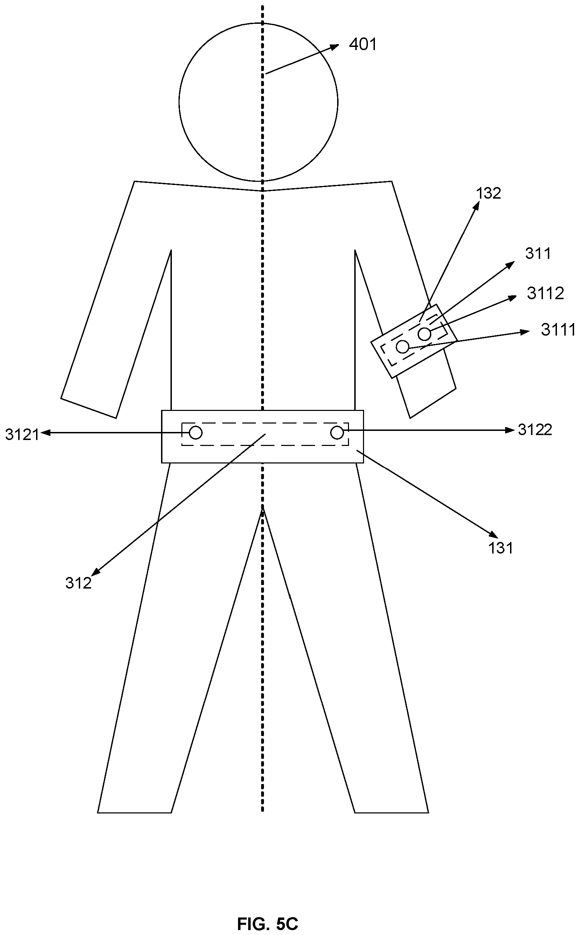

The processing circuit 320 refers to a circuit component for processing the signals from the human body (e.g., extracting the electrocardio signal) collected by the electrocardio channel. In some embodiments, the processing circuit 320 may include a low-pass filter circuit and/or a high-pass filter circuit. In some embodiments, the processing circuit 320 may be integrated into the processing device 110. More descriptions regarding the processing circuit may be found in FIG.1 and FIG.5C and related descriptions thereof.

In some embodiments, the processing circuit 320 may extract the electrocardio signal based on the signals from the body collected by the at least two sets of electrocardio channels 310.

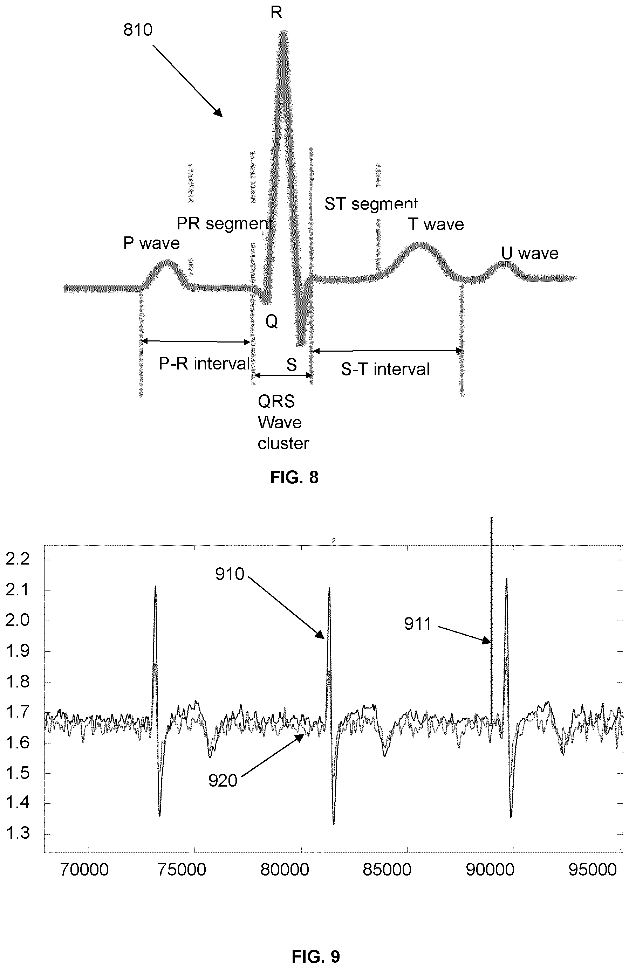

The processing circuit 320 may extract the electrocardio signal based on the signals from the human body collected by the at least two sets of electrocardio channels 310 in a plurality of ways. In some embodiments, the processing circuit 320 extracts the electrocardio signals based on the signals from the human body collected by the at least two sets of electrocardio channels 310, which includes separately extracting characteristic points of the signals from the human body collected by each of the at least two sets of electrocardio channels 310, matching characteristic points of the signals from the human body collected by the at least two sets of electrocardio channels 310, and designating signals corresponding to matched characteristic points of the at least two sets of electrocardio channels 310 as the electrocardio signal. Detailed descriptions regarding the characteristic points may be found elsewhere in the present disclosure, e.g., FIG.8-FIG.12 and related descriptions thereof.

In some embodiments, because the electrocardio signal is less affected by a collection position (i.e., the distribution position of the electrocardio channel) and the noise signal (i.e., the electromyographic signal and the motion artifact) is more affected by the acquisition position, among the plurality of signals from the human body collected by the at least two sets of electrocardio channels 310 at different distribution positions, a component that has a high level of synchronicity and correlation is the electrocardio signal, and a component that has a low level of synchronicity and correlation is the noise signal. Therefore, at least two sets of electrocardio channels 310 with different distribution positions (the electrodes are located at different positions of the human body to form at least two sets of electrocardio channels with different distribution positions) are used to measure signals at different positions of the human body. Then, the processing circuit 320 is configured to extract a signal component that is synchronized and correlated among the plurality of signals collected by the at least two sets of electrocardio channels 310 as the electrocardio signal. Thus, an effective identification of the electrocardio signals is ensured, to avoid the interference of the noise signal.

As shown in FIG. 3B-3C, the at least two sets of electrocardio channels 310 may include a first electrocardio channel 311 and a second electrocardio channel 312. In some embodiments, the at least two sets of electrocardio channels 310 may also include other electrocardio channels, such as the Nth electrocardio channel 31N.

As shown in FIG. 3C, the first electrocardio channel 311 may include two electrocardio electrodes, i.e., a first electrode 3111 and a second electrode 3112. The second electrocardio channel 312 may include two electrocardio electrodes, i.e., a third electrode 3121 and a fourth electrode 3122. In FIG. 2, the electrocardio channel AB may be designated as a first electrocardio channel, then the electrode A and the electrode B may be designated as a first electrode and a second electrode, respectively. The electrocardio channel CD may be designated as a second electrocardio channel, then the electrode C and the electrode D may be designated as a third electrode and a fourth electrode, respectively. More descriptions regarding the third electrode and the fourth electrode may be founf in related descriptions hereinafter.

It may be understood that electrodes (e.g., the electrocardio electrode) of the first electrocardio channel 311 and the second electrocardio channel 312 are independent of each other at this time.

In some embodiments, for electrocardio electrodes in the at least two sets of electrocardio channels 310, electrodes included in the electrocardio channels (e.g., the first electrode 3111 and the second electrode 3112 included in the first electrocardio channel 311, or the third electrode 3121 and the fourth electrode 3122 included in the second electrocardio channel 312) may be affixed to the human skin in the form of a flexible patch. The patch has an open-ended shape and may be a regular shape such as a circle, an oval, a rectangle, or other irregular shapes. In some embodiments, the shape of the patch may be determined based on actual application requirements. For example, the shape of the patch may be determined based on the shape of the bone under the skin of the lower back where the electrode is affixed.

For the electrocardio electrodes in the at least two sets of electrocardio channels 310, the electrodes included in the electrocardio channels are of unlimited materials. In some embodiments, the electrodes included in the electrocardio channels may be electrodes made of a single material, such as a metal fabric electrode, a conductive silicon electrode, a hydrogel electrode, a metal electrode, or the like. Preferably, the electrodes included in the electrocardio channel may be metal fabric electrodes and conductive silicon electrodes. Further preferably, the electrodes included in the electrocardio channel may be metal fabric electrodes, which are less resistive, have lower impedance, and lower contact impedance with the skin. The smaller the contact impedance between the electrodes included in the electrocardio channels and the skin is favorable for reducing the interference of the motion artifact with the electrocardio signal collected by the at least two sets of electrocardio channels 310.

In some embodiments, for the electrocardio electrodes in the at least two sets of electrocardio channels 310, the electrodes included in the electrocardio channel may protrude from a surface of the wearable body 130, providing a pre-pressure for the electrodes included in the electrocardio channel. It is beneficial for the electrodes included in the electrocardio channel to fully adhere to the human skin and accurately collect the electrocardiogram signal. In some embodiments, a height of an electrode included in an electrocardio channel protruding relative to the surface of the wearable body 130 depends on a thickness of the electrode included in the electrocardio channel. The thickness of the electrode included in the electrocardio channel may be a dimension of the electrode included in the electrocardio channel in a direction perpendicular to the surface of the wearable body 130. In some embodiments, the thickness of the electrode included in the electrocardio channel is related to the material. For example, in the case of collecting the electrocardio signal using the metal fabric electrode, a thickness of the metal fabric electrode may be in a range of 10 µm to 5 mm. Preferably, the thickness of the metal fabric electrode may be in a range of 100 µm to 3 mm. Further preferably, the thickness of the metal fabric electrode may be in a range of 500 µm to 2 mm.

In some embodiments, for the electrocardio electrodes in the at least two sets of electrocardio channels 310, the electrodes included in the electrocardio channels may also be electrodes formed by superposition of different materials. For example, electrodes composed of a metal fabric material and a conductive silicon material, which not only have low contact impedance between their contact and skin but also have advantages such as being skin-friendly, washable, friction resistant, etc., avoiding discomfort to the human body brought about by the electrodes in contact with the skin.

In some embodiments, the electrodes included in the electrocardio channel may be of the same or different sizes. For example, the sizes may be 1cm x 1cm, 1cm x 2cm, 1cm x 3cm, 1cm x 4cm, 1cm x 5cm, 2cm x 2cm, 2cm x 3cm, 2cm x 4cm, 2cm x 5cm, 3cm x 3cm, 3cm x 4cm, 3cm x 5cm, 4cm x 4cm, 4cm x 5cm, or 5cm x 5cm. It should be noted that theelectrodes included in the electrocardio channel are not limited to the sizes listed above, but may also be other sizes. In some embodiments, the electrodes of the above sizes may be rectangular structures or rectangular structures that have been chamfered (e.g., rounded or beveled).

As shown in FIG. 2, a size of each electrode included in the electrocardio channels in a first extending direction a (e.g., a size a1 of the electrocardio electrode and a size a2 of the reference electrode) or the size of the electrode included in a second extending direction b (e.g., a size b1 of the electrocardio electrode and a size b of the reference electrode) may be as large as possible without exceeding a size of the wearable body 130 in the first extending direction a or the sizes of the electrodes included in the second extending direction b. Therefore, the electrodes included in the electrocardio channel may be ensured to have a large fitting area with the skin, to reduce the contact impedance between the electrodes included in the electrocardio channel and the skin to which they are adhered, thus reducing the interference of the motion artifact on the electrocardio signal. At the same time, a larger size of each electrode included in the electrocardio channels in the first extending direction a or in the second extending direction b may make the electrode included in the electrocardio channel less susceptible to deformation and deflection during wearing, which is conducive to reducing the motion artifact and improving the quality of the electrocardio signal. In addition, the larger size of each electrode included in the electrocardio channels in the first extending direction a or the larger size of each electrode included in the electrocardio channels in the second extending direction b may ensure that, in case of a plurality of motions or creases, the electrodes included in the electrocardio channels do not fall off from the skin and affect the acquisition of the electrocardio signal.

In some embodiments, the size of each electrode included in the electrocardio channels in the first extending direction a or the size of each electrode included in the electrocardio channel in the second extending direction b may refer to a maximum size of each electrode included in the electrocardio channel in the first extending direction a or the size of a maximum size of each electrode included in the electrocardio channel in the second extending direction b, respectively. In some embodiments, the sizes of the electrodes included in the electrocardio channel in the first extending direction a or the sizes of the electrodes included in the electrocardio channel in the second extending direction b may be in a range of 5 mm to 100 mm. In some embodiments, the sizes of the electrodes included in the electrocardio channel in the first extending direction a or the sizes of the electrodes included in the electrocardio channel in the second extending direction b may be in a range of 10 mm to 45 mm. In some embodiments, the sizes of the electrodes included in the electrocardio channel in the first extending direction a or the sizes of the electrodes included in the electrocardio channel in the second extending direction b may be in a range of 15 mm to 40 mm. In some embodiments, the sizes of the electrodes included in the electrocardio channel in the first extending direction a or the sizes of the electrodes included in the electrocardio channel in the second extending direction b may be in a range of 20 mm to 30 mm.

In some embodiments, the first extending direction a refers to a circumferential direction in which the wearable body 130 is worn by the human body at the lower back of the human body when the wearable body 130 (e.g., a strap-like structure) is worn. The second extending direction b refers to a direction perpendicular to the first extending direction a. To enable the electrodes included in the electrocardio channels to fit more closely with the lower back of the human body to prevent the electrodes from being significantly displaced relative to the skin of the lower back of the human body during the movement of the human body, in some embodiments, the sizes of the electrodes included in the electrocardio channels in the first extending direction a is larger than the size of the electrodes included in the electrocardio channels in the second extending direction b.

As shown in FIG. 2, a distance d between two electrocardio electrodes (e.g., the electrode A and the electrode B) corresponding to each set of electrocardio channels may not be less than a certain value or may be as large as possible, to avoid the weak electrocardio signal collected due to the distance d being excessively small, which sufficiently ensures a strength of the electrocardio signal collected by the electrocardio channels, to facilitate the extraction of the electrocardio signals in the subsequent process. The distance between the two electrocardio electrodes may refer to a central distance between the two electrocardio electrodes. In some embodiments, a specific value of the distance between the two electrocardio electrodes corresponding to each set of electrocardio channels may be determined based on experiments, simulations, or the like.

In some embodiments of the present disclosure, by setting at least two electrodes corresponding to each set of electrocardio channels (each set of electrocardio channels includes at least two electrodes, i.e., the electrodes are independent of each other) at different distribution positions, a noise signal with a large variability may be obtained, which facilitates the subsequent rapid separation and extraction of the electrocardio signal. Further, the acquisition of the signals from the human body based on the plruality of different sets of electrocardio channels may further help to identify the electrocardio signal and thus improve the quality of electrocardio signal.

In some embodiments, the at least two sets of electrocardio channels 310 may include a first electrocardio channel 311 and a second electrocardio channel 312. The first electrocardio channel 311 may include a first electrode and a second electrode, and the second electrocardio channel 312 may include a first electrode and a third electrode. In FIG. 2, the electrocardio channel AB may be designated as the first electrocardio channel, and the electrode A and the electrode B may be designated as the first electrode and the second electrode, respectively. The electrocardio channel AC may be designated as the second electrocardio channel, and the electrode C may be designated as the third electrode.

It may be understood that a common electrode (e.g., a first electrode) exists at this time between electrodes of the first electrocardio channel 311 and the second electrocardio channel 312.

As shown in FIG. 2, two-by-two combinations between the electrodes may be performed to form different electrocardio channels. For example, any two electrodes between the electrode A, the electrode B, the electrode C, and the electrode D (the electrode E is the reference electrode) may be combined two-by-two to form six different electrocardio channels. Exemplarily, the combination of the electrode A and the electrode B forms an electrocardio channel AB; the combination of the electrode A and the electrode C forms an electrocardio channel AC; the combination of the electrode A and the electrode D forms an electrocardio channel AD; the combination of the electrode B and the electrode C forms an electrocardio channel BC; the combination of the electrode B and the electrode D forms an electrocardio channel BD; and the electrode C and the electrode D forms an electrocardio channel CD. It is to be noted that, in practical application scenarios, the combination of a plurality of electrodes is not limited to the combination formation of any two of the above-mentioned electrodes. The combination of the electrodes is formed, and the combination manner between the electrodes may be selected according to the specific situation. Merely by way of example, in some embodiments, the electrode A, the electrode B, the electrode C, and the electrode D may be selectively combined two by two to form two sets of electrocardio channels. For example, the combination of the electrode A and the electrode B forms the electrocardio channel AB, and the combination of the electrode C and the electrode D forms the electrocardio channel CD. The electrocardio signals collected between electrocardio channels formed by other combinations of electrodes (e.g., the electrocardio channels AD, BD, AC, BC, etc.) may be calculated by a potential difference between different electrodes.

In some embodiments, a common electrode (e.g., the first electrode) exists between the electrodes of the first electrocardio channel 311 and the second electrocardio channel 312, at which point the second electrode and the third electrode may be located at different positions on the human body for acquiring the signals from the human body. For example, the electrocardio monitoring system has the electrode A, the electrode B, and the electrode C. The combination of the electrode A and the electrode B forms the electrocardio channel AB, and the combination of the electrode A and the electrode C forms the electrocardio channel AC. At this time, the electrode A is the common electrode for the two sets of electrocardio channels.

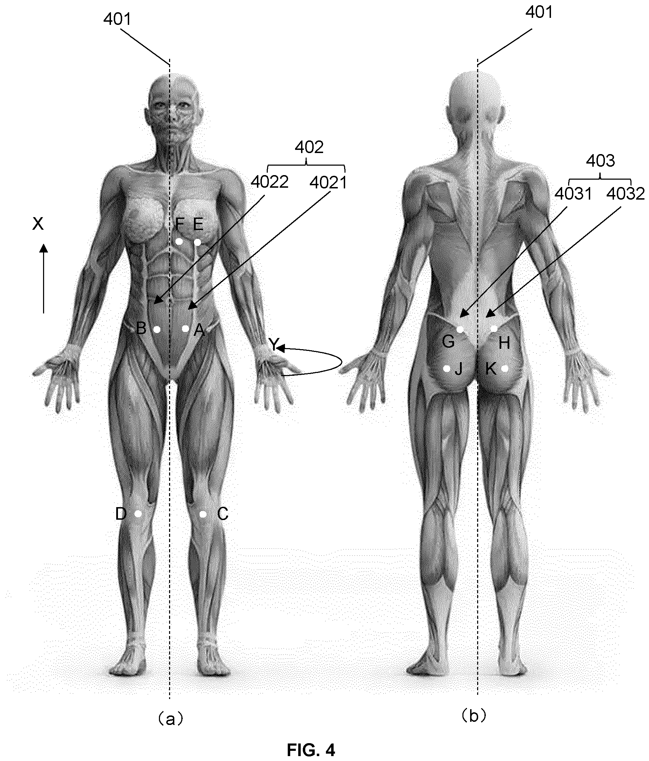

FIG. 4 is a schematic diagram illustrating a whole human body according to some embodiments of the present disclosure. FIG. 5A is a schematic diagram illustrating a structure of a wearable body according to some embodiments of the present disclosure. FIG. 5B is a schematic diagram illustrating a structure of a wearable body according to some other embodiments of the present disclosure.

In some embodiments, as shown in FIG.4 - FIG.5B, the first electrode 3111 and the second electrode 3112 may be located on two sides of a median sagittal plane of the human body, and the third electrode 3121 and the fourth electrode 3122 may likewise be located on the two sides of the median sagittal plane of the human body.

As shown in FIG. 4, the median sagittal plane of the human body refers to a plane that passes through a median line 401 of the human body and divides the human body into two equal or approximate parts. The median line 401 of the human body may be determined based on a line from the tip of the nose of the human body to the middle of the two nipples, from the middle of the two nipples to the middle of the umbilicus of the abdomen, or from the middle of the umbilicus of the abdomen to the middle of the joint of the pubic bone.

In some embodiments, each set of electrocardio channels may include at least two electrodes, and the at least two electrodes may include the first electrode 3111 and the second electrode 3112. The first electrode 3111 and the second electrode 3112 may also be located on two sides of a human body axis through the heart when standing upright, where the human body axis is parallel to the median line 401 of the human body hereinabove. By arranging the first electrode 3111 and the second electrode 3112 on the two sides of the human body axis that passes through the heart, a distance between the first electrode 3111 and the second electrode 3112 is made to be large, ensuring that the collected electrocardio signal is strong.

In some embodiments, when the human body wears the wearable body, in a vertical direction (e.g., a direction perpendicular to the ground when the human body is standing, the direction directed by the arrow X shown in FIG. 4 (a)), the first electrode 3111 and the second electrode 3112 are affixed to a position between the iliac bones to the chest on each side of the median sagittal plane of the human body. For example, the first electrode 3111 is located in a region between the left iliac bone and the left chest, and the second electrode is located in a region between the right iliac bone and the right chest. In an extending direction of the waist circumference of the human body (e.g., the direction indicated by the arrow Y shown in FIG. 4(a)), the first electrode 3111 and the second electrode 3112 are respectively affixed to the iliac bones on both sides of the median sagittal plane of the human body, and the affixed size does not exceed 1/6 of the waist circumference of the human body. When the human body wears the wearable body, the first electrode 3111 and the second electrode 3112 are respectively located at a position of the left lower back 4031 and the right lower back 4032, or the left abdominal region 4021 and the right abdominal 4022 of the human body. At this time, the first electrode 3111 and the second electrode 3112 are located on two sides of the median sagittal plane of the human body, ensuring that the collected electrocardio signal is strong.

As shown in FIGs. 4-5B, when the human body wears the wearable body 130, the first electrode 3111 and the second electrode 3112, or the third electrode 3121 and the fourth electrode 3122, may be affixed to the skin at the lower back and on each side of the median sagittal plane of the human body, respectively. The lower back of the human body may be a region between a lower end of the rib cage to a lower end of the ilium bone of the human body, which mainly includes the abdominal and the iliac bones. The median sagittal plane of the human body may divide the lower back of the human body into a left lower back and a right lower back. In some embodiments, the first electrode 3111 and the second electrode 3112, or the third electrode 3121 and the fourth electrode 3122, may be affixed to the skin of the left lower back and the right lower back, respectively. In some embodiments, the first electrode 3111 and the second electrode 3112, or the third electrode 3121 and the fourth electrode 3122, may be affixed to the skin of the left ankle and the right ankle, respectively. It may be understood that the first electrode 3111 and the second electrode 3112, or the third electrode 3121 and the fourth electrode 3122, may also be affixed to other parts of the human body, respectively. For example, the skin of the left thigh and the right thigh, the skin of the left calf and the right calf, or the like. In some embodiments, the first electrode 3111 and the second electrode 3112, or the third electrode 3121 and the fourth electrode 3122, may be arranged symmetrically about the median sagittal plane of the human body, which allows a better consistency in motion artifacts at the position of the first electrode 3111 and the second electrode 3112, or the third electrode 3121 and the fourth electrode 3122 to be affixed to. Thus, it is favorable to eliminating the motion artifacts to improve the quality of the electrocardio signal. In addition, symmetrically setting the electrodes corresponding to the electrocardio channels about the median sagittal plane of the human body facilitates a user to understand the placement of the electrodes when wearing the wearable body, facilitates the user to wear the wearable body, and is in line with an intuition of the user when wearing the wearable body, thereby improving usability of the electrocardio monitoring system.

It should be noted that the first electrode 3111 and the second electrode 3112, or the third electrode 3121 and the fourth electrode 3122 may also be asymmetrically arranged about the median sagittal plane of the human body. For example, when the user wears the wearable body 130, the first electrode 3111 is located at the abdomen on one side of the median sagittal plane of the human body, and the second electrode 3112 is located at the iliac bone on the other side of the median sagittal plane of the human body. As another example, when the user wears the wearable body 130, the third electrode 3121 is located at the abdomen on one side of the median sagittal plane of the human body, and the fourth electrode 3122 is located at the lower back region on the other side of the median sagittal plane of the human body. As yet another example, the first electrode 3111 (electrode E in FIG. 4) and the second electrode 3112 (electrode F in FIG. 4) may both be located on the chest on one side of the median sagittal plane of the human body. The electrodes corresponding to the electrocardio channels are arranged asymmetrically about the median sagittal plane of the human body, and the user may adjust the position of the electrodes according to an experience when wearing the wearable body so that the electrodes are adjusted to a region with high comfort. At the same time, the diversity of distribution positions of the electrodes may also improve the applicability of the electrocardio monitoring system, to meet the needs of different types of users (e.g., body size, age, gender, dominant hand, etc.) and facilitate the user to place the electrodes.

As shown in FIG. 4, when the human body wears the wearable body 130, the first electrode 3111 and the second electrode 3112 may be affixed to the skin of the abdomen 402 on two sides of the median sagittal plane of the human body, respectively. The third electrode 3121 and the fourth electrode 3122 may be affixed to the skin of the knee joint on two sides of the median sagittal plane of the human body, respectively. In some embodiments, the median sagittal plane of the human body divides the abdomen 402 into the left abdomen 4021 and the right abdomen 4022. When the human body wears the wearable body 130, the first electrode 3111 (electrode A in FIG. 4) and the second electrode 3112 (electrode B in FIG. 4) may be affixed to the skin of the left abdomen 4021 and the skin of the right abdomen 4022, respectively. The third electrode 3121 (electrode C in FIG. C in FIG. 4) and a fourth electrode 3122 (electrode D in FIG. 4) may be affixed to the skin of the left knee joint and the skin of the right knee joint on two sides of the median sagittal plane of the human body, respectively. Thus, a plurality of signals from the human body are collected. In some embodiments, when the human body is wearing the wearable body 130, the first electrode 3111 and the second electrode 3112 are in a position where the first electrode 3111 and the second electrode 3112 are affixed to the skin of the left abdomen 4021 and the right abdomen 4022, respectively. The third electrode 3121 and the fourth electrode 3122 are in a position where the third electrode 3121 and the fourth electrode 3122 are affixed to the skin of the left knee joint and the right knee joint, respectively, which may be symmetrical about the median sagittal plane of the human body. Therefore, the consistency of the motion artifacts at the positions to which the first electrode 3111 and the second electrode 3112, or the third electrode 3121 and the fourth electrode 3122 are affixed may be improved, thereby facilitating the elimination of the motion artifacts to improve the quality of the electrocardio signal. At the same time, the distribution of the electrodes corresponding to the electrocardio channels on two sides of the median sagittal plane of the human body ensures that the electrocardio signal collected by the electrocardio channel has a high intensity, improving the quality of the collected electrocardio signal. In addition, the abdomen and the knee joints of the human body are less sensitive, and setting the electrodes of the electrocardio channels in these positions may make the user have a better wearing experience when wearing the device. It should be noted that the position where the first electrode 3111 and the second electrode 3112 are affixed to the skin of the left abdomen 4021 and the right abdomen 4022, respectively, and the position where the third electrode 3121 and the fourth electrode 3122 are affixed to the skin of the left knee joint and the right knee joint, respectively, may also be asymmetric about the median sagittal plane of the human body.

As shown in FIG. 4, when the human body wears the wearable body 130, the first electrode 3111 and the second electrode 3112 may be affixed to the skin of the lower back 403 on two sides in the median sagittal plane of the human body, respectively, and the third electrode 3121 and the fourth electrodes 3122 may be affixed to the skin of the buttock on two sides of the median sagittal plane of the human body, respectively. In some embodiments, the median sagittal plane of the human body may divide the hindquarters 403 of the human body into the left lower back 4031 and the right lower back 4032. When the human body wears the wearable body 130, the first electrode 3111 (electrode G in FIG. 4) and the second electrode 3112 (electrode H in FIG. 4) may be affixed to the skin of the left lower back 4031 and the right lower back 4032, respectively, and the third electrode 3121 (electrode J in FIG. 4) and fourth electrode 3122 (electrode K in FIG. 4) may be affixed to the left hip and the right hip, respectively, to collect a plurality of signals from the human body. In some embodiments, when the human body is wearing the wearable body 130, the position where the first electrode 3111 and the second electrode 3112 are affixed to the skin of the left lower back 4031 and the right lower back 4032, respectively, and the position where the third electrode 3121 and fourth electrode 3122 are affixed to the skin of the left hip and the right hip, respectively, may be symmetrical about the median sagittal plane of the human body. Therefore, the consistency of the motion artifacts at the positions where the first electrode 3111 and the second electrode 3112, and the third electrode 3121 and the fourth electrode 3122 are affixed may be improved, thereby facilitating the elimination of the motion artifacts to improve the quality of the electrocardio signal. The electrodes corresponding to the electrocardio channels are distributed at the left lower back 4031 and the right lower back 4032 in the median sagittal plane of the human body, which ensures that the electrocardio signal collected by the electrocardio channels have a higher intensity and improves the quality of the collected electrocardio signal. Meanwhile, since the human body has less muscle in the lower back region, distributing the electrodes at the lower back region of the human body may reduce the interference of the electromyographic signal with the electrocardio signal. In addition, when the user is ill or disabled and only lies on his back most of the time, setting the electrodes in the lower back region and the buttocks may reduce a relative movement of the electrodes with the skin of the human body and reduces the motion artifacts to improve the quality of the electrocardio signal. It is noted that, the position where the first electrode 3111 and the second electrode 3112 affixed to the skin of the left lower back 4031 and the right lower back 4032, respectively, and the position where the third electrode 3121 and the fourth electrode 3122 affixed to the skin of the left hip and the right hip, respectively, may also be asymmetric about the median sagittal plane of the human body.

In some embodiments, by maintaining the consistency between the first electrode 3111 and the second electrode 3112, and the third electrode 3121 and the fourth electrode 3122, the consistency between the motion artifacts of the positions where the first electrode 3111 and the second electrode 3112, and the third electrode 3121 and the fourth electrode 3122 are affixed, which is more conducive to the elimination of the motion artifacts, to make the electrocardio signal have a higher quality. In some embodiments, the first electrode 3111 and the second electrode 3112, and the third electrode 3121 and the fourth electrode 3122 being consistent may include a material, a size (e.g., a size in the first extending direction, a size in the second extending direction, and a thickness, etc.), pressure on the skin, etc., or a combination thereof. In some embodiments, through elastic consistency in part of the first electrode 3111 and the second electrode 3112, and the third electrode 3121 and the fourth electrode 3122 carried by the wearable body 130, consistency of the motion artifacts at the positions where the first electrode 3111 and the second electrode 3112, and the third electrode 3121 and the fourth electrode 3122 are affixed may be made the better, so as to facilitate the elimination of the motion artifacts and improve the quality of the electrocardio signal. It is to be noted that the material, size, pressure on the skin, etc., of the first electrode 3111 and the second electrode 3112, and the third electrode 3121 and the fourth electrode 3122, may be consistent or inconsistent.

In some embodiments of the present disclosure, by setting two electrocardio electrodes (e.g., the first electrode and the second electrode) corresponding to each set of electrocardio channels on two sides of the median sagittal plane of the human body, respectively, and preferably, setting the two electrocardio electrodes symmetrically about the median sagittal plane of the human body, not only the distance between the two electrocardio electrodes may be ensured, but also the strength of the electrocardio signals may be ensured. Further, the consistency of the position of the first electrode and the second electrode are affixed to may be improved, thereby facilitating the elimination of the motion artifacts and improving the quality of the electrocardio signal.

In some embodiments, a central distance between two electrodes corresponding to different electrocardio channels is not less than 5 mm along the first extending direction a of the at least two electrodes.

In some embodiments, the two electrodes corresponding to the different electrocardio channels may be understood as two electrocardio electrodes corresponding to the different electrocardio channels. The central distance between the two electrodes corresponding to the different electrocardio channels along the first extending direction a of the at least two electrodes may be understood to be a distance between geometric centers of the two electrocardio electrodes corresponding to the different electrocardio channels in the first extending direction a. As shown in FIG. 2, the two electrocardio electrodes corresponding to the electrocardio channel AB are the electrode A and the electrode B, and the two electrocardio electrodes corresponding to the electrocardio channel CD are the electrode C and the electrode D. The central distance between the two electrodes may be a central distance between the electrode A and the electrode C, a central distance between the electrode A and the electrode D, a central distance between the electrode B and the electrode C, and a central distance between the electrode B and the electrode D, in the first extending direction a.

Limiting the central distance between two electrodes corresponding to different electrocardio channels in the first extending direction a may make the electromyographic signal included in signals collected by the different electrocardio channels different, so that the extraction of the electrocardio signal based on a level of synchronicity and a correlation may be effective to avoid extracting the electromyographic signal as the electrocardio signal, which is conducive to ensuring the accuracy of the electrocardio signal. In order to improve a difference in electrocardio signals collected by different electrocardio channels, in some embodiments, the central distance between the two electrodes corresponding to the different electrocardio channels is not less than 5 mm. To further improve the difference in the electrocardio signals collected by the different electrocardio channels, in some embodiments, the central distance between the two electrodes corresponding to the different electrocardio channels is not less than 10 mm. In some embodiments, the central distance between the two electrodes corresponding to the different electrocardio channels is not greater than 100 mm to make the overall size of the electrocardio monitoring system or the wearable body smaller, which is easy for the user to wear and carry.

FIG. 5C is a schematic diagram illustrating a structure of a wearable body according to some other embodiments of the present disclosure.

As shown in FIG.5A-FIG.5C, at least two sets of electrocardio channels 310 may be provided on the same wearable body 130.

The wearable body 130 may be configured to carry two electrocardio electrodes corresponding to each set of electrocardio channels (e.g., a first electrode and a second electrode corresponding to a first electrocardio channel, and a third electrode and a fourth electrode corresponding to a second electrocardio channel), and affix the two electrocardio electrodes corresponding to each set of electrocardio channels to a human body (e.g., to two sides of a median sagittal plane of the human body), respectively. The wearable body 130 may be in a plurality of forms.

In some embodiments, the wearable body 130 may be in a form of pants (e.g., shorts, pants, dress pants, etc.) shown in 5A, and the first electrode 3111 and the second electrode 3112, and the third electrode 3121 and the fourth electrode 3122 may be provided inside the waist of the pants at intervals along a circumferential direction of the waist of the pants. Thus, when the human body wears the pants, the first electrode 3111 and the second electrode 3112, and the third electrode 3121 and the fourth electrode 3122 at the waist of the pants, may be located on two sides of the median sagittal plane of the human body, respectively. For example, the first electrode 3111 and the second electrode 3112, or the third electrode 3121 and the fourth electrode 3122 may be provided at positions such as the left waist side and the right waist side, the left hip and the right hip, the left knee joint and the right knee joint, and the left ankle and the right ankle, respectively (as shown by the dashed rectangle in FIG. 5A). In some embodiments, the wearable body 130 may also be in the form of a skirt, and the first electrode 3111 and the second electrode 3112, and the third electrode 3121 and the fourth electrode 3122 may be provided at an inner side of the waist of the skirt at intervals along the circumferential direction of the skirt waist of the skirt, so that the first electrode 3111 and the second electrode 3112, the third electrode 3121 and the fourth electrode 3122 may be located on two sides of the median sagittal plane of the human body, respectively.

In some embodiments, the wearable body 130 may also be a belt structure with elasticity, and the first electrode 3111 and the second electrode 3112, and the third electrode 3121 and the fourth electrode 3122 may be provided on the belt structure at intervals along an extending direction (or referred to as the length direction) of the belt structure. Therefore, the user may attach the belt structure, or fix the belt structure through a buckle member, to the lower back of the user, so that the first electrode 3111 and the second electrode 3112, the third electrode 3121 and the fourth electrode 3122 may be located on two sides of the median sagittal plane of the human body. In some embodiments, the wearable body 130 may be in the form of a waistband as shown in FIG. 5B, and the first electrode 3111 and the second electrode 3112, and the third electrode 3121 and the fourth electrode 3122 may be provided at intervals on a surface of the waistband that is affixed to the human skin. In some embodiments, two ends of the waistband may be combined using, for example, Velcro patches or snaps, to facilitate the user to wear or take off.

In some embodiments of the present disclosure, by designing the wearable body as a band structure with elasticity, the user may be assured of better wearing comfort and resistance to washing. In addition, by setting at least two electrodes corresponding to each set of electrocardio channels on the same wearable body, it is more convenient for the user to wear the wearable body and the detection of the electrocardio signal.

In some embodiments, the at least two sets of electrocardio channels 310 may be in fixed connection with the wearable body 130. The fixed connection may include a non-detachable connection (e.g., glued connection, etc.) and a detachable connection (e.g., snap connection, inlay connection, etc.). In some embodiments, the wearable body 130 is the belt structure or the pants, and the side of the two electrocardio electrodes corresponding to each set of electrocardio channels (e.g., the first electrode and the second electrode corresponding to the first electrocardio channel, and the third electrode and the fourth electrode corresponding to the second electrocardio channel) which is not in contact with the human skin with the belt structure or the pants may be connected to the wearable body 130 through bonding, snap-fitting, inlaying, or the like. Exemplarily, when the user needs to measure the electrocardio signal, the two electrocardio electrodes corresponding to each set of electrocardio channels are provided on the belt structure, a waist, ends of legs of the pants, the middle of the legs of the pants, and the hip position based on detachable connection. The positions of two electrocardio electrodes corresponding to each set of electrocardio channels in the wearable body 130 may also be adjusted according to the body shape (e.g., height, weight, waist circumference, etc.) of the user to apply to users with different body shapes. When the user needs to clean the wearable body 130, the components such as the two electrocardio electrodes corresponding to each set of electrocardio channels may be removed from the wearable body 130 to prevent the wearable body 130 from damaging other components such as the two electrocardio electrodes corresponding to each set of electrocardio channels during the cleaning process of the wearable body 130.

In some embodiments, a first Velcro patch may be provided on a side of the wearable body 130 (e.g., the belt structure or the waist of the pants) that is affixed to the user, the first Velcro patch may be distributed along the extending direction of the belt structure or the circumference of the waist of the pants. A second Velcro patch is provided on a side where the two electrocardio electrodes corresponding to each set of electrocardio channels are back away from a side in contact with the user's body. The second Velcro patch may be adhesively bonded with the first Velcro patch to realize that the two electrocardio electrodes corresponding to each set of electrocardio channels are detachably connected with the wearable body 130. In some embodiments, the first Velcro patch may cover a side of the wearable body 130 that is affixed to the user. When the first Velcro patch is an overall structure, it may interfere with theelasticity of the wearable body 130, resulting in a poor wearing experience for the user. In some embodiments, there may be a plurality of first Velcro patches, and the plurality of first Velcro patches are sequentially spliced together on the wearable body 130 on the side that is affixed to the user. At this time, a gap between the plurality of first Velcro patches may ensure the wearable body 130 to maintain its own elasticity. Meanwhile, it is also convenient to adjust the positions of the two electrocardio electrodes corresponding to each set of electrocardio channels in the wearable body 130, so as to be applicable to users of different body sizes. In some embodiments, the plurality of first Velcro patches may also be arranged at intervals at a circumferential side along the extending direction of the belt structure or the position of the electrode. For example, each first Velcro patch may have a length of 4 cm and a distance of 1 cm between two adjacent first Velcro patches. In some embodiments, the first Velcro patch or the second Velcro patch may be made of a non-woven material, which is a softer material. In such a case, when a user wears the wearable body 130, the first Velcro patch does not cause discomfort to the user when the skin of the lower back comes into contact with the first Velcro patch, thereby improving the user experience when the user wears the wearable body 130.

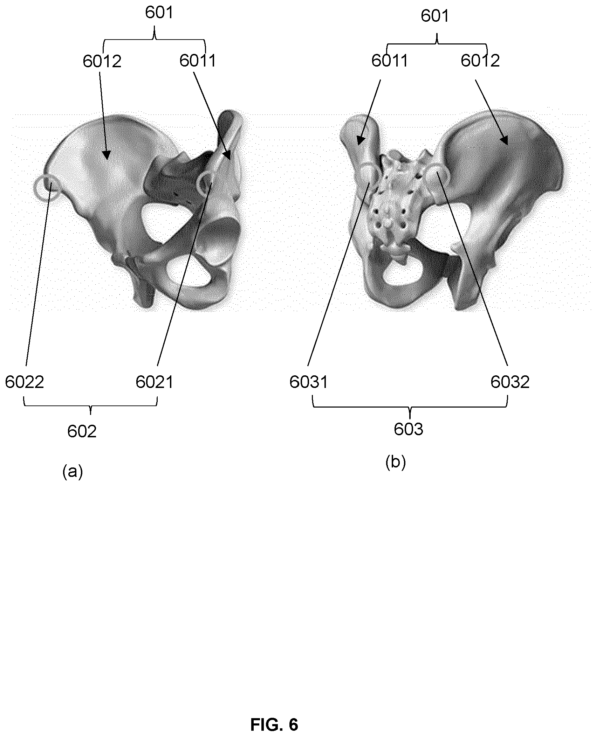

FIG. 6 is a schematic diagram illustrating an ilium bone at the lower back of a human body according to some embodiments of the present disclosure.

In some embodiments, at least two electrodes corresponding to one set of at least two sets of electrocardio channels 310 may be located at the iliac bones on two sides of a median sagittal plane of the human body, and at least two electrodes corresponding to another set of the at least two sets of electrocardio channels 310 may be located at the lower back and on the two sides the median sagittal plane of the human body, respectively.

As shown in FIG. 6, the median sagittal plane of the human body may divide iliac bones 601 into a left iliac bone 6011 and a right iliac bone 6012. At least two electrodes (e.g., the first electrode and the second electrode) corresponding to one set of the at least two sets of electrocardio channels 310 may be affixed to the skin regions covering the left iliac bone 6011 and the right iliac bone 6012, respectively, and at least two electrodes (e.g., the third electrode and the fourth electrode) corresponding to another set of electrocardio channels may be affixed to the skin covering the left lower back 4031 and the right lower back 4032, respectively, to collect the electrocardio signals of the human body. In some embodiments, when the human body wears the wearable body 130, positions of the first electrode 3111 and the second electrode 3112 affixed to the skin regions covering the left ilium bone 6011 and the right ilium bone 6012, respectively, and positions of the third electrode 3121 and the fourth electrode 3122 affixed to the skin covering the left lower back 4031 and the right lower back 4032, respectively, may be symmetrical about the median sagittal plane of the human body. Therefore, the consistency of motion artifacts at affixed positions of the first electrode 3111 and the second electrode 3112, and of the third electrode 3121 and the fourth electrode 3122, may be improved, thus facilitating the elimination of the motion artifacts and improving the quality of the electrocardio signal. In some embodiments, the positions at which the first electrode 3111 and the second electrode 3112 are affixed to the skin regions covering the left ilium bone 6011 and the right ilium bone 6012, respectively, and the positions the third electrode 3121 and the fourth electrodes 3122 affixed to the skin covering the left lower back 4031 and the right lower back 4032, respectively, may also be asymmetric about the median sagittal plane of the human body.

Further, as shown in FIG. 6(a), when the human body wears the wearable body 130, at least two electrodes (e.g., the first electrode and the second electrode) corresponding to one set of the at least two sets of electrocardio channels 310 may be affixed to the anterior superior iliac spine 602 on the two sides of the median sagittal plane of the human body, respectively. The anterior superior iliac spine 602 may include a left anterior superior iliac spine 6021 and a right anterior superior iliac spine 6022 disposed on the left ilium 6011 and the right ilium 6012, respectively. Exemplarily, when the human body wears the wearable body 130, the first electrode 3111 and the second electrode 3112 may be affixed to skin regions covering the left anterior superior iliac spine 6021 and the right anterior superior iliac spine 6022, respectively, to collect the electrocardio signal of the human body. Because there are fewer muscles at the left anterior superior iliac spine 6021 and the right anterior superior iliac spine 6022, the electromyographic signal interferes less with the electrocardio signal collected by the first electrode 3111 and the second electrode 3112, even when the human body is in motion, and thus a better quality of the electrocardio signal may be ensured. The left anterior superior iliac spine 6021 and the right anterior superior iliac spine 6022 are symmetrical with respect to the median sagittal plane of the human body, and the motion artifacts corresponding to the first electrode 3111 and the second electrode 3112 are in a better consistency, which is conducive to the elimination of the motion artifacts and improving the quality of the electrocardio signal. Meanwhile, a difference between a distance from the left anterior superior iliac spine 6021 to the heart and a distance from the right anterior superior iliac spine 6022 to the heart is large, and a potential difference between the two is also large. The first electrode 3111 and the second electrode 3112 are affixed to the skin regions covering the left anterior superior iliac spine 6011 and the right anterior superior iliac spine 6011, respectively, which may improve a strength of a collected electrocardio signal. In addition, the left anterior superior iliac spine 6021 and the right anterior superior iliac spine 6022 are provided with bumps to be sufficiently affixed by the first electrode 3111 and the second electrode 3112, ensuring that the first electrode 3111 and the second electrodes 3112 are not easily dislodged. Further, the human body is less sensitive to the corresponding skin regions at the left anterior superior iliac spine 6021 and the right anterior superior iliac spine 6022, which ensures that the human body has a better wearing comfort when wearing the wearable body 130.

Similarly, the first electrode 3111 and the second electrode 3112 may be affixed to a posterior superior iliac spine 603 on the two sides of the median sagittal plane of the human body, respectively, as shown in FIG. 6(b). The posterior superior iliac spine 603 may include a left posterior superior iliac spine 6031 located on the left ilium bone 6011 and a right posterior superior iliac spine 6032 located on the right ilium bone 6012, respectively. Exemplarily, when the human body wears the wearable body 130, the first electrode 3111 and the second electrode 3112 may be affixed to the skin regions covering the left posterior superior iliac spine 6031 and the right posterior superior iliac spine 6032, respectively, to collect the electrocardio signal of the human body. Due to the lack of muscles at the left posterior superior iliac spine 6031 and the right posterior superior iliac spine 6032, even though the human body is in motion, the electromyographic signal interferes less with the electrocardio signal collected by the first electrode 3111 and the second electrode 3112, which ensures that the electromyographic signal has a better quality. The left posterior superior iliac spine 6031 and the right posterior superior iliac spine 6032 are symmetrical with respect to the median sagittal plane of the human body, and the motion artifacts have a better consistency, which is conducive to eliminating the motion artifacts, improving the quality of the electromyographic signal.

Correspondingly, since there are more muscles at the lower back 403 of the human body as compared to that at the anterior superior iliac spine 602, the electrocardio signal collected by the third electrode 3121 and the fourth electrode 3122 is more strongly interfered with the electrocardio signal. Thus, a difference between the electromyographic signal collected by the third electrode 3121 and the fourth electrode 3122 and the electromyographic signal collected by the first electrode 3111 and the second electrode 3112 may be more obvious, which is more favorable to the separation and extraction of the electromyographic signal.

In some embodiments of the present disclosure, by affixing the at least two electrodes corresponding to one set of the at least two sets of electrocardio channels to the iliac bones on the two sides of the median sagittal plane of the human body, respectively, the electrocardio signal may be ensured to have a better quality, and the motion artifacts may be ensured to have a better consistency, which is conducive to the elimination of the motion artifacts, improveing the quality of the electrocardio signal. By affixing the at least two electrodes corresponding to another set of electrocardio channels in the at least two sets of electrocardio channels to the lower back on the two sides of the median sagittal plane of the human body, which may make the differences between the electromyographic signals and motion artifacts collected by one set of the at least two sets of electrocardio channels and another set of electrocardio channels based on at least two electrodes more obvious, thus more favorable to the separation and extraction of the electrocardio signals.

In some embodiments, when the wearable body 130 is worn by the human body, in a vertical direction (e.g., a direction perpendicular to the ground when the human body is standing, the direction indicated by the arrow X shown in FIG. 4(a)), the first electrode 3111 and the second electrode 3112 corresponding to the first electrocardio channel 311 are affixed to a position between the iliac bone and the chest on the two sides of the median sagittal plane of the human body, respectively. For example, the first electrode 3111 is located in a region between the left ilium bone and the left chest, and the second electrode is located in a region between the right ilium bone and the right chest. Because the iliac bone is more prominent relative to the rest of the human body, the electrodes do not fit well with the iliac bone. When the human body is in motion, a relative motion between the electrodes and the iliac bone is prone to occur, resulting in too severe motion artifacts of the electrocardio signal of the iliac bones collected by the electrocardio channel. In the vertical direction, a position below the iliac bones is far away from the heart, and the strength of the collected electrocardio signal is not high. Furthermore, considering that when the electrodes of the electrocardio channel are set at the chest, the user's experience and comfort when wearing the electrocardio channel are poor. In addition, there are more muscles in the chest, and the electrocardio signal collected by the electrocardio channel are seriously affected by the electromyographic signal. In such a case, the electrode corresponding to the electrocardio channel is herein set in a region between the ilium bones and the chest, and the electrode is close to the heart, which may improve the strength of the electrocardio signal collected by the electrocardio channel. At the same time, the electrode is more likely to fully affixed to the region between the ilium bones and the chest. There are fewer muscles in the region, which may reduce the motion artifacts and interference of the electromyographic signal. In some embodiments, in an extending direction (e.g., the direction indicated by the arrow Y shown in FIG. 4(a)) of the waist circumference of the human body, a size of the iliac bones on the two sides of the median sagittal plane of the human body where the first electrode 3111 and the second electrode 3112 affixed, respectively, do not measure more than 1/6 of the waist circumference of the human body. Setting the electrodes of the electrocardio channel near the iliac bones on the two sides of the median sagittal plane of the human body allows for a larger distance between the two electrodes to ensure that the collected electrocardio signal is stronger.

It is to be noted that another set of electrocardio channels in the at least two sets of electrocardio channels 310 may also not be set at the lower back and on the two sides of the median sagittal plane of the human body, as long as there existing a significant difference between the electromyographic signal collected by at least two electrodes corresponding to one set of electrocardio channels (e.g., the first electrode and the second electrode), respectively, and the electromyographic signal collected by at least two electrodes corresponding to another set of electrocardio channels (e.g., the third electrode and the fourth electrode) of the at least two sets of electrocardio channels 310, respectively. Exemplarily, another set of electrocardio channels in the at least two sets of electrocardio channels 310 may also be provided in the abdomen and legs (e.g., knees, etc.) on the two side of the median sagittal plane of the human body.

In some embodiments, the at least two sets of electrocardio channels 310 may be provided on different wearable bodies 130.

In some embodiments, the wearable body 130 may also be in the form of a combination of detachable wearable members as illustrated in FIG. 5C. For example, the wearable body 130 may be a combination of a belt 131 and a wrist protector 132, a combination of pants (e.g., shorts, pants, dress pants, etc.) and the wrist protector 132, a combination of a top and the wrist protector 132, or the like. In some embodiments, when the at least two sets of electrocardio channels 310 include a first electrocardio channel 311 and a second electrocardio channel 312, based on the fact that the wearable body 130 is the combination of detachable wearable members, two electrocardio electrodes (e.g., the first electrode 3111 and the second electrode 3112) corresponding to the first electrocardio channel 311 may be provided at intervals along a circumferential direction on one of the wearable members (e.g., the wrist protector 132), and two electrocardio electrodes (e.g., the third electrode 3121 and the fourth electrode 3122) corresponding to the second electrocardio channel 312 are provided at intervals along the circumferential direction on the other wearable member (e.g., the belt 131). In some embodiments, when the at least two sets of electrocardio channels 310 include three sets of electrocardio channels (e.g., a first electrocardio channel, a second electrocardio channel, and a third electrocardio channel), based on the fact that the wearable body 130 is the combination of detachable wearable members, two of the three sets of electrocardio channels (e.g., the first electrocardio channel and the second electrocardio channel) may be provided on one of the wearable members (e.g., the top), and one of the three sets of electrocardio channels (e.g., the third electrocardio channel) may be provided on another wearable member (e.g., the pants).

In some embodiments of the present disclosure, by further designing the wearable body as the combination of detachable wearable members, the difference between electromyographic signals and motion artifacts in a plurality of different sets of electrocardio channels may be better realized, which is conducive to the improve the quality of the electrocardio signal. In addition, by setting the wearable body into the combination of detachable wearable members, the distribution position of electrodes corresponding to the electrocardio channels may be adjusted according to the needs of different users, to facilitate the wearing of the user and improve the user's wearing comfort.

In some embodiments, the processing circuit 320 may include a low-pass filter circuit, and a cutoff frequency of the low-pass filter circuit is no less than 100 Hz (e.g., 150 Hz, 200 Hz, 250 Hz, etc.). Preferably, the cutoff frequency of the low-pass filter circuit may also be no less than 200 Hz (e.g., 300 Hz, 500 Hz, etc.). Further preferably, the cutoff frequency of the low-pass filter circuit may be no less than 400 Hz and no greater than 0.5 times a sampling frequency.

The low-pass filter circuit refers to a signal processing device that serves to filter out high-frequency components from an input signal, retaining only low-frequency components. In some embodiments, processing a plurality of signals from the human body collected by at least two electrodes corresponding to each set of electrocardio channels based on the cutoff frequency of the low-pass filter circuit to filter out the interference of the electrocardio signal caused by high-frequency noise, which is convenient for subsequent extraction of the electrocardio signal. By setting the cutoff frequency in this range, it also facilitates anti-aliasing when sampling and saves computational resources. According to Nyquist's Law of Sampling, the sampling frequency should be at least two times the target frequency to collect the signal, otherwise, it may be aliased to form noise. In addition, it is not possible to increase the sampling frequency indefinitely because of the consumption of resources and because Bluetooth transmission does not support high-speed transmission. In the embodiment of the present disclosure, the signals from the human body are filtered using low-pass filters, unneeded high-frequency portions are filtered out, and then an ADC chip is utilized to perform adc collection, thus avoiding high sampling frequency.

A frequency of the electromyographic signal is in a range of 10Hz to 1000Hz, and the frequency of the electromyographic signal is more concentrated in a range of 20Hz to 400Hz. A frequency of the electrocardio signal is in a range of 0.05Hz to 100Hz, and the frequency of the electrocardio signal is more concentrated in a range of 0.25Hz to 45Hz. In some embodiments when the cutoff frequency of the low-pass filter circuit is not lower than 100Hz (e.g., 150Hz, 200Hz, 250Hz, etc.), the electrocardio signals of a plurality of different electrocardio channels may be retained after processing by the low-pass filter circuit. At the same time, some of the electromyographic signals are filtered out to reduce the interference of the noise signal. However, filtering out part of the electromyographic signal may result in less difference between the remaining electromyographic signals of the plurality of sets of different electrocardio channels, or even between the electromyographic signals and the electrocardio signals of the plurality of sets of different electrocardio channels, which is unfavorable for the subsequent extraction of the electrocardio signals. Understandably, when the cutoff frequency of the low-pass filter circuit is closer to 100 Hz, the difference between the electromyographic signals and the electrocardio signals of the plurality of sets of different electrocardio channels becomes smaller, which is less favorable to the subsequent extraction of the electrocardio signals. In some embodiments, when the cutoff frequency of the low-pass filter circuit is not lower than 400 Hz (e.g., 450 Hz, 500 Hz, etc.), the electromyographic signals and the electrocardio signals of the plurality of sets of different electrocardio channels may be retained after being processed by the low-pass filter circuit, and the difference between the electromyographic signals of the plurality of sets of different electrocardio channels remain essentially unaffected. Whereas other high-frequency noises may be filtered out, thereby facilitating the subsequent extraction of the electrocardio signals. It should be noted that the cutoff frequency of the low-pass filter circuit is not limited to the above-described not less than 100 Hz, but may also be other ranges. In some embodiments, the cutoff frequency of the low-pass filter circuit may also be not less than 50 Hz, at which time a higher-order low-pass filter circuit may be used for filtering the collected signal. Specifically, in some embodiments, the 40 dB cutoff frequency of the low-pass filter circuit is not lower than 50 Hz. Preferably, the 20 dB cutoff of the low-pass filter circuit is not lower than 50 Hz.

In some embodiments, a weight element 210 may be provided on an electrode corresponding to one of at least two sets of electrocardio channels 310, as shown in FIG. 2.

The weight element 210 refers to an object having a certain mass. The structure and material of the weight element 210 are not limited, for example, the weight element 210 may be a copper piece, an iron ring, or the like. In some embodiments, a size of the weight element 210 may be determined based on sizes of the at least two electrodes corresponding to each set of electrocardio channels, the material of the weight element 210, or the like. More descriptions regarding the weight element may be found hereinafter.

In some embodiments, the wearable body 130 may be a structure with elasticity (e.g., the belt structure), the at least two sets of electrocardio channels 310 and the wearable body 130 may be fixedly connected, and the weight element 210 may be provided between the electrodes corresponding to one set of the at least two sets of electrocardio channels 310 and the wearable body 130. As shown in FIG. 2, the weight element 210 is provided between the electrode C corresponding to the electrocardio channel CD and the wearable body 130.

In some embodiments, the weight element 210 may be provided on a side of the wearable body 130 that is back away from the electrodes corresponding to one set of the at least two sets of electrocardio channels 310. In some embodiments, the weight element 210 may also be provided on the wearable body 130. Distances between the weight element 210 and the electrodes corresponding to one set of electrocardio channels of the at least two sets of electrocardio channels 310 satisfy a predetermined distance condition. The predetermined distance condition may be that the distance does not exceed a distance threshold, and the distance threshold may be a predetermined value, an empirical value, or an experimental value, etc.

In some embodiments, the weight element 210 may be set up in other ways, which are not limited in the present disclosure.

In some embodiments of the present disclosure, by providing the weight element on an electrode corresponding to one of at least two sets of electrocardio channels, when the human body wears the wearable body and the human body changes from a state of motion to a stationary state, the electrode provided with the weight element has a larger inertia relative to the electrode that is not provided with the weight element, and the electrode may continue to move. Based on the fact that the wearable body is of a structure with elasticity, the movement of the electrode that is provided with the weight element is pulled back to its original position only after a certain time. In such a case, the motion artifacts captured by the electrodes with the weight element and those captured by the electrodes without the weight element significantly different in the time domain (i.e., the motion artifacts of different electrocardio channels are significantly different in the time domain, and the motion artifacts captured by the electrodes with configurations have a hysteresis). Therefore, it is conducive to the identification of the motion artifacts and the electrocardio signal and the elimination of the motion artifacts, to improve the quality of the electrocardio signal.

In some embodiments, a difference between the motion artifacts of different electrocardio channels may also be improved by adjusting factors related to electrode quality such as the size, material, shape, and other factors of electrodes corresponding to one set of the at least two sets of electrocardio channels 310. In some embodiments, the difference between motion artifacts of the different electrocardio channels may also be improved by adjusting friction coefficients of the electrodes corresponding to one set of electrocardio channels of the at least two sets of electrocardio channels 310 that is affixed to a side of the human skin.

In some embodiments, the weight element 210 may include at least a portion of a circuit structure of the processing circuit 320. By integrating at least a portion of the circuit structure of the processing circuit 320 on the weight element 210, a volume of the electrocardio monitoring system 300 may be reduced, and it may also be possible to enable one set of the electrocardio channels of the at least two electrocardio channels is increased, thereby increasing the difference between the motion artifacts of different electrocardio channels, which in turn facilitates the elimination of the motion artifacts to improve the quality of the electrocardio signal. The difference between the motion artifacts of the different electrocardio channels is not obvious when a mass of the weight element 210 is too small, and when the mass of the weight element 210 is too large the experience of the user when wearing the weight element 210 is poor. Based on this, in some embodiments, the mass of the weight element 210 may be in a range of 10g to 200g, so that the difference between the motion artifacts of the different electrocardio channels is increased, which in turn facilitates the elimination of the motion artifacts to improve the quality of the electrocardio signal while ensuring the experience of the user when wearing the device.

In some embodiments, the electrocardio monitoring system may further include a power source that provides power to the circuit structure, the circuit structure being electrically connected to the power source. Exemplarily, the power source may include any one or more of a lithium battery, a storage battery, a dry cell battery, or the like.

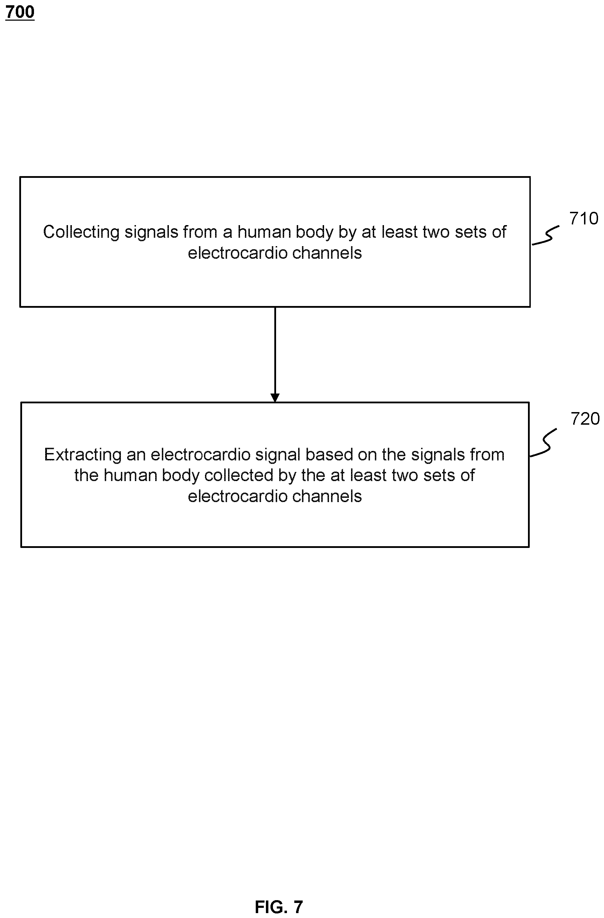

FIG. 7 is a flowchart illustrating an exemplary process of measuring an electrocardio signal according to some embodiments of the present disclosure. As shown in FIG. 7, a process 700 includes the following operations. In some embodiments, the process 700 may be performed by the processing device 110 (e.g., the processing circuit 320).

In 710, signals from a human body are collected by at least two sets of electrocardio channels.

In some embodiments, the signals from the human body collected by the at least two sets of electrocardio channels (e.g., at least two sets of electrocardio channels 310) may include an electrocardio signal and a noise signal, each set of the electrocardio channels includes at least two electrodes, and the at least two sets of the electrocardio channels collect the signals from the human body through the at least two electrodes, respectively. The at least two electrodes may be configured to affixed to the skin of the human body to collect the signals from the human body.

For example, in some embodiments, the at least two electrodes of the set of electrocardio channels may include a first electrode and a second electrode, and the first electrode and the second electrode may be affixed to different parts of the human body. A site to which the first electrode is affixed may have a first potential, and a site to which the second electrode is affixed may have a second potential. A difference between the first potential and the second potential may be used to reflect the electrocardio signal and the noise signal from the human body. Detailed descriptions regarding the electrocardio channels and the corresponding electrodes, and the collection of the signals from the human body by the at least two sets of electrocardio channels may be found in FIG.1-FIG.6 and related descriptions thereof.

In some embodiments, after the signals from the human body is collected by the electrocardio channel, the signals from the human body may be preprocessed, which may in turn allow for extraction of the electrocardio signal based on the preprocessed signals from the human body. In some embodiments, the pre-processing may include filtering, noise reduction, and the like, which is conducive to subsequent signal processing.

In some embodiments, the electrocardio signals are extracted, based on the signals from the human body collected by the at least two sets of electrocardio channels, by a processing circuit (e.g., processing circuit 320). The processing circuit may include a low-pass filter circuit, and the low-pass filter circuit includes a cutoff frequency of not lower than 100 Hz. Preferably, the cutoff frequency of the low-pass filter circuit is not lower than 400 Hz. Further preferably, the cutoff frequency of the low-pass filter circuit is not lower than 400 Hz. The electrocardio signals of the plurality of different electrocardio channels after processing by the low-pass filter circuit are basically retained, and the difference between the electromyographic signals of the plurality of different electrocardio channels is unaffected, whereas other high-frequency signals are filtered out, thus facilitating subsequent extraction of the electrocardio signal. Descriptions regarding the processing circuit and the low-pass filter circuit may be found in FIG.1-FIG.3C and FIG.5A and related descriptions thereof.

In 720, the electrocardio signal is extracted based on the signals from the human body collected by the at least two sets of electrocardio channels.

In some embodiments, extracting the electrocardio signal based on the signals from the human body collected by the at least two sets of electrocardio channels may include extracting the electrocardio signal based on electrocardio signals and the noise signal from the human body collected by the at least two sets of electrocardio channels. In some embodiments, signal components with higher synchronicity and correlation among the plurality of signals from the human body collected by the at least two sets of electrocardio channels at different distribution positions may be extracted as the electrocardio signal, and signal components with lower synchronicity and correlation may be determined as the noise signal. Accordingly, a true electrocardio signal may be efficiently extracted from the collected signals from the human body, and the interference of the noise signal may be excluded. Descriptions regarding extracting the electrocardio signal from the signals from the human body collected by the at least two sets of electrocardio channels may be found in FIG.8-FIG.13 and related descriptions thereof.

FIG. 8 is a schematic diagram illustrating a standardized waveform of an electrocardio signal according to some embodiments of the present disclosure. As shown in FIG. 8, an electrocardio wave 810 has the characteristics of P-R interval, QRS wave cluster, S-T interval, and U wave, in which the P-R interval has P wave and PR segment, the QRS wave cluster has trough Q, R wave and trough S, and the S-T interval has ST segment and T wave. Ideally, the electrocardio signal including the electrocardio wave 810 without noise signal interference may be collected by at least two electrodes of the electrocardio channel. However, affected by the fact that the human body muscles corresponding to the distribution positions of the electrodes are different, the muscles have different firing degrees, and the skin contact resistance is different, etc., different electrocardio channels may correspondingly generate a signal with a difference, i.e., the noise signal, and the noise signal may affect the waveform expression of the electrocardio signal collected at the same time.

FIG. 9 is a diagram illustrating a waveform of signals from a human body in a stationary position captured by an electrocardio channel AB and an electrocardio channel CD (as shown in FIG. 2) according to some embodiments of the present disclosure. At this point, the electrocardio channel AB may be distributed at, for example, a hipbone position of the human body. Specifically, two electrodes corresponding to the electrocardio channel AB are affixed to the hipbones on two sides of the human body. The electrocardio channel CD may be distributed on, for example, the back of the human body. Specifically, the two electrodes corresponding to the electrocardio channel CD are affixed to the left and right sides of the back, respectively.

As shown in FIG.9, curve 910 and curve 920 are waveform curves of the signals from the human body in the standing state collected by the electrocardio channel AB and the electrocardio channel CD, respectively. As the human body is in a stationary state, the muscles are less involved, myoelectric in the collected signals from the human body is less involved, i.e., there are fewer noise signals. The presented waveform curves are closer to intrinsic waveform curves of the electrocardio signal, such as standard electrocardio signal waveforms of FIG.8. Since characteristics about the QRS wave cluster in curves 910 and 920 shown in FIG.9 are most significant, to facilitate the extraction of the electrocardio signal, the present disclosure may utilize the characteristics of the QRS wave cluster for the extraction of the electrocardio signals. In addition, since the electrocardio signals collected by the electrocardio channel AB and the electrocardio channel CD show synchronization and high correlation, the overlap and consistency of the curve 910 and the curve 920 are high. However, the noise signal affects the waveform curves presented by the collected signals from the human body, causing them to differ from the standard electrocardio signal waveform. For example, the process of the electrocardio channel AB collecting the human body signals is affected by the noise signal, causing the curve 910 to have a third wave peak 911 that is inconsistent with the curve 920.

Continuing to refer to FIG. 9, a magnitude of the curve 910 in a direction of a signal strength expressed in a vertical coordinate is greater than a magnitude of the curve 920 in the vertical coordinate, it may be seen that the signal strength of the signal collected by the electrocardio channel AB is greater than the signal strength of the signal collected by the electrocardio channel CD. The curve 910 almost coincides with the curve 920 in the time indicated by a horizontal coordinate, and there is a subtle difference. It may be known that the signal collected by the electrocardio channel AB is approximately the same in time as the signal collected by the electrocardio channel CD. Thus, it may be seen that the signals from the human body collected by different electrocardio channels are close in time, but there may be differences in the signal strength.

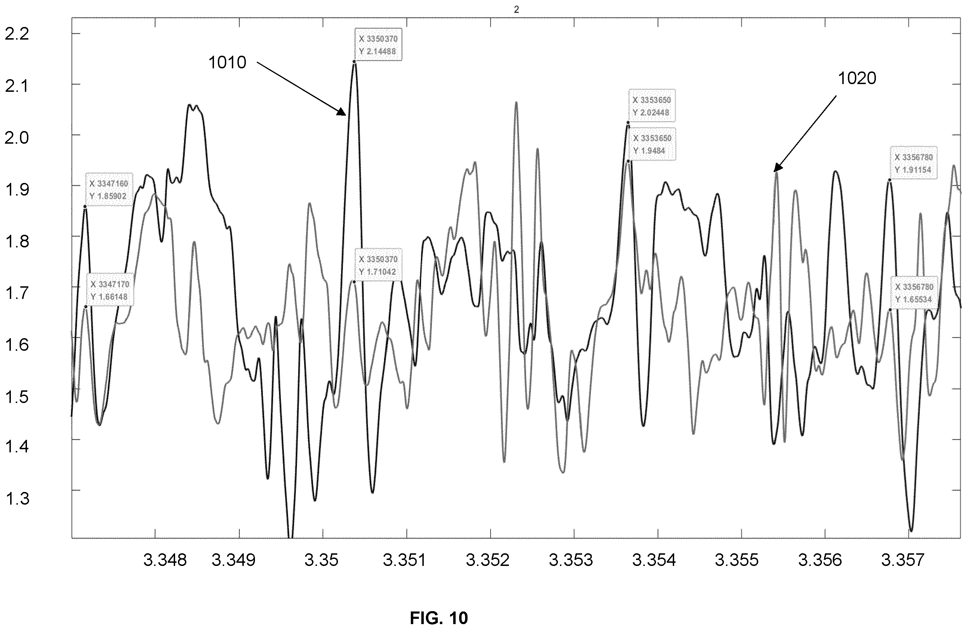

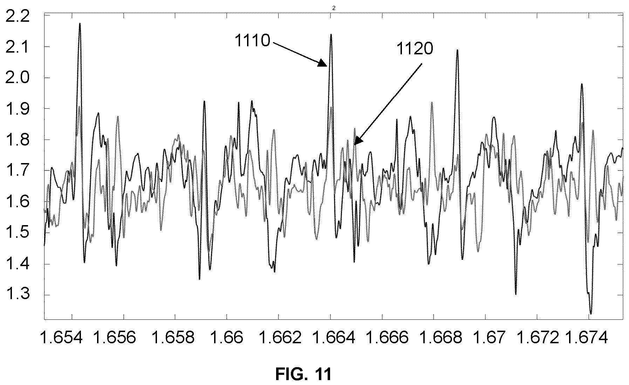

FIG. 10 is a diagram illustrating signals from the human body under an accelerated running state collected by the electrocardio channel AB and the electrocardio channel CD according to some embodiments of the present disclosure. In FIG.10 and FIG.11, the horizontal coordinates denote the time, and the vertical coordinates denote the signal strength. FIG. 11 is a diagram illustrating signals from the human body under an accelerated running state collected by the electrocardio channel AB and the electrocardio channel CD according to some embodiments of the present disclosure. The horizontal coordinates in FIG.10 and FIG.11 denote the time and the vertical coordinates denote the signal strength.

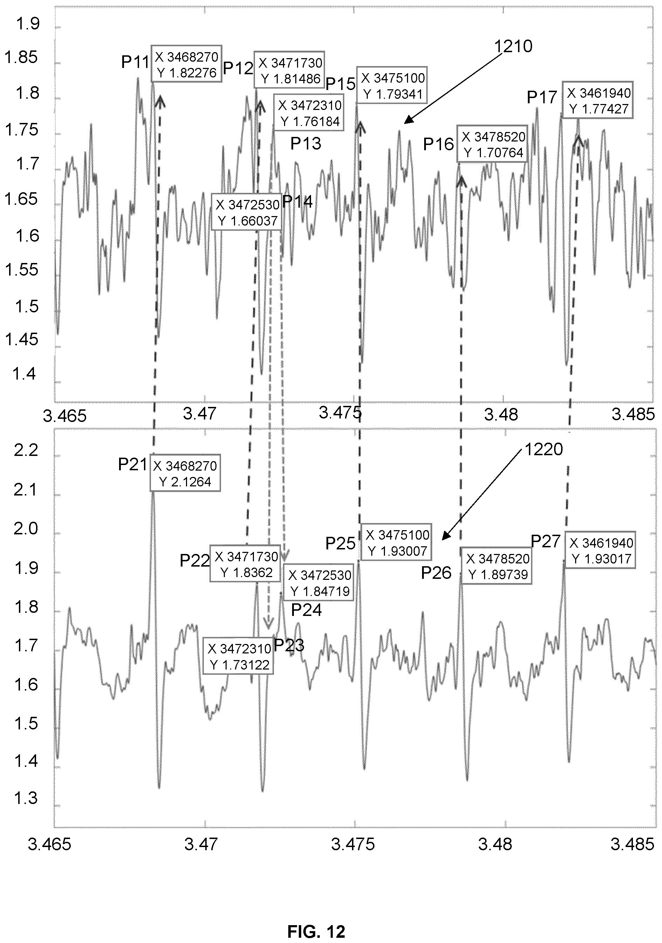

As shown in FIG.10, curve 1010 and curve 1020 are one kind of waveform curves of the signals from the human body under an accelerated running state collected by the electrocardio channel AB and the electrocardio channel CD respectively. As shown in FIG.11, curve 1110 and curve 1120 are another kind of waveform curves of the signals from the human body under the accelerated running state collected by the electrocardio channel AB and electrocardio channel CD, respectively. When the human body is in motion, the muscles are more involved, and the myoelectric and motion artifacts are more involved in the collected signals from the human body, i.e., there are more noise signals. Thus, the characteristics of the electrocardio waveform presented by each set of electrocardio channels, including the P-R interval, the QRS wave cluster, the S-T interval, the U wave, etc., are less obvious. Due to the different distribution positions of the electrocardio channel AB and electrocardio channel CD, which are differently affected by the myoelectric and the motion artifacts, resulting in a large difference in the noise signals collected by the electrocardio channel AB and the electrocardio channel CD and leading to a difference in the signals from the human body collected by the electrocardio channel AB and the electrocardio channel CD. However, referring to FIG.10 and FIG.11, curve 1010 and curve 1020, as well as curve 1110 and curve 1120, have part of peaks or valleys of the waveforms that overlap in the time domain or have partial correspondence agreement within a range of a very small time difference, which is caused by the synchronization and high correlation of the electrocardio signals collected by different electrocardio channels. More descriptions regarding the high correlation of the electrocardio signals collected by the electrocardio channel AB and the electrocardio channel CD may be found in FIG.12.

FIG. 12 is a diagram illustrating human body signals collected by the electrocardio channel AB and the electrocardio channel CD according to some embodiments of the present disclosure. In FIG.12, the horizontal coordinate denotes the time and the vertical coordinate denotes the signal strength.

As shown in FIG.12, curve 1210 and curve 1220 are the waveform curves of the signals from the human body collected by the electrocardio channel AB and the electrocardio channel CD, respectively, Due to the more noise signals, characteristics of the electrocardio waves presented by the curves are not obvious. The electrocardio signals in the signals from the human body collected by the electrocardio channel AB and the electrocardio channel CD have a high correlation, which is represented in the curve 1210 and the curve 1220, specifically by their synchronized corresponding wave peaks along the time axis. Characteristics of the QRS wave cluster corresponding to the electrocardio wave near wave peaks are indicated by dashed line connections in FIG. 12, and the wave peaks are actually the R peaks of the electrocardio wave. At the same time, there is a small difference in the time corresponding to the synchronized wave peaks in the curve 1210 and the curve 1220.

Accordingly, as described in FIGs. 8-FIG. 12, in some embodiments, the characteristics of synchronization and high correlation of the electrocardio signals collected by different electrocardio channels may be utilized. By identifying characteristic points in the signals from the human body collected by the electrocardio channel AB and the electrocardio channel CD that include electrocardio wave information, respectively, and matching the characteristic points in the signals from the human body collected by the electrocardio channel AB and the electrocardio channel CD, correlation components corresponding to the matched characteristic points (e.g., characteristic points of the electrocardio channel AB and characteristic points of the electrocardio channel CD within a time difference) in the signals from the human body collected by the electrocardio channel AB and the electrocardio channel CD are extracted as the electrocardio signals to exclude the interference of the noise signal. The characteristic points may include the P-R interval, the QRS wave cluster, the S-T interval, the U-wave, and the trough Q, the R wave, and the trough S in the QRS wave cluster, or the like. Preferably, the characteristic points may include any one or more of a shape of the R wave, a strength of the R wave, a shape of the trough S, a shape of a Q wave, an R-S interval, or the like, in the QRS wave cluster. In some embodiments, the time difference may be in a range of 0 ms to 4 ms. Preferably, the time difference may be in a range of 0 ms to 2 ms. Preferably, the time difference may also be in a range of 0 ms to 1 ms.

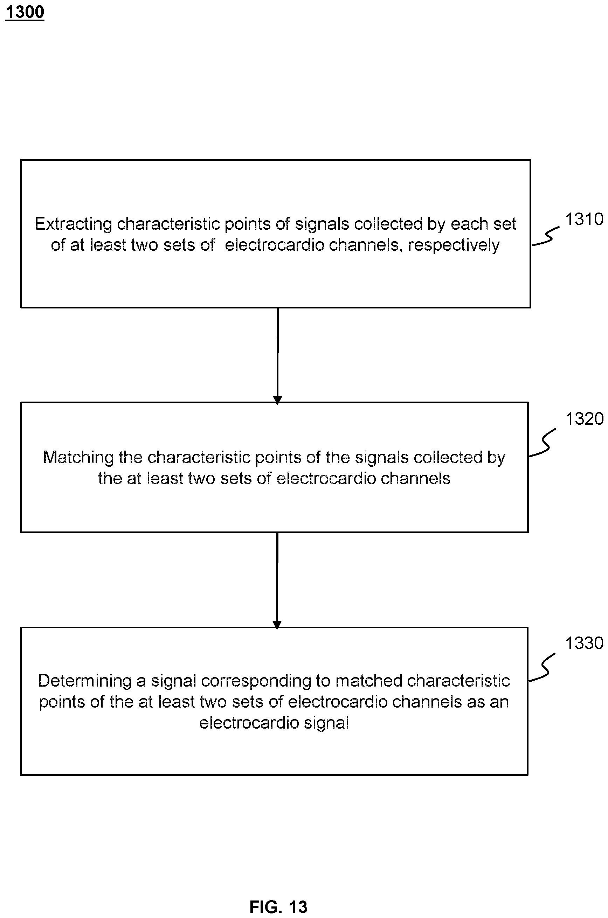

FIG. 13 is a flowchart illustrating an exemplary process of extracting an electrocardio signal according to some embodiments of the present disclosure. As shown in FIG. 13, a process 1300 includes the following operations. In some embodiments, the process 1300 may be performed by the processing device 110 (e.g., the processing circuit 320).

In 1310, characteristic points of signals collected by each set of at least two sets of electrocardio channels are extracted, respectively.

A characteristic point of a signal may be a point with a specific characteristic in a signal from the human body collected through an electrocardio channel. In some embodiments, the characteristic point may include poles on the curve of the signal from the human body. In some embodiments, the characteristic point may be a point on or near the P-R interval, the QRS wave cluster, the S-T interval, or the U wave as shown in FIG. 8. For example, the characteristic point is a point on a left side near the P-R interval. In some embodiments, the characteristic point may include poles on or near the P-R interval, the QRS wave cluster, the S-T interval, or the U-wave as shown in FIG. 8. For example, the characteristic point are poles of the T wave in the S-T interval. In some embodiments, the characteristic points may include poles and the R-S interval in the QRS wave cluster shown in FIG. 8. For example, the characteristic point is a Q point (the lowest point of the trough Q), an R point (the highest point of the R wave), or an S point (the lowest point of the trough S). In some embodiments, the characteristic point may be any one or more of the shapes of the R wave shape, the strength of the R wave, the shape of the trough S, the shape of the Q wave, the R-S interval, or the like, in the QRS wave cluster shown in FIG. 8.

In some embodiments, the characteristic point may be extracted based on mathematical morphology, wavelet algorithms, differential thresholding, etc. Taking extracting the characteristic point R peak based on the difference thresholding algorithm as an example, a part of collected signals from the human body with larger slopes is highlighted by a difference operation, most of which is the QRS wave cluster of the electrocardio signal. A slope threshold is set, and a part exceeding the slope threshold is screened out from the part with a larger slope, most of which is the QRS wave cluster of the electrocardio signal. The R peak is detected in the part exceeding the slope threshold, i.e., the R peak is extracted as the characteristic point. In some embodiments, one or more characteristic points of the signals collected by each set of electrocardio channels may be extracted, for example, extracting the R points of the signals collected by each set of electrocardio channels, or extracting the R points, Q points, and S points of the signals collected by each set of electrocardio channels.

In 1320, the characteristic points of the signals collected by the at least two sets of electrocardio channels are matched.

In some embodiments, the signals collected by the at least two sets of electrocardio channels may be matched based on one type of characteristic point, and the one type of characteristic point may include one or more characteristic points of the same type. For example, the R point serves as a characteristic point, the signals collected by the at least two sets of electrocardio channels are matched based on the R point. For example, as shown in FIG. 12, P11, P12, P13, P15, P16, and P17 shown in FIG. 12 are R points extracted from the signals collected by the electrocardio channel AB, FIG. 12 P21, P22, P24, P25, P26, P27 shown in FIG.12 are the R points extracted from the signals collected by the electrocardio channel CD, as follows.

**Comparison table of coordinates of characteristic points-Table 1**

| Curve 1210 | Curve 1220 |
|---|---|
| P11(3468270,1.82276) | P21 (3468270,2.1264) |
| P12(3471730,1.81486) | P22(3471730,1.8362) |
| P13(3472310,1.76184) | - |
| - | P24(3472530,1.84719) |
| P15(3475100,1.79341) | P25(3475100,1.93007) |
| P16(3478520,1.70764) | P26(3478520,1.89739) |
| P17(3461940,1.77427) | P27(3461940,1.93017) |

As seen from Table 1, P11, P12, P15, P16, and P17 are matched one-to-one with P21, P22, P25, P26, and P27 within an allowable time difference. Continuing to refer to FIG. 12, P13 on the curve 1210 corresponds to P23 on the curve 1220 (3472310,1.73122), and P23 is not an extreme point of the R wave in the curve 1220. Therefore, P13 on the curve 1210 cannot form a match with the curve 1220. P24 on the curve 1220 corresponds to P14 on the curve 1210 (3472530, 1.66037), and P14 is not an extreme point of the R wave in the curve 1210. Therefore, P14 on the curve 1220 cannot form a match with curve 1210. Therefore, the characteristic points of the signals collected by the electrocardio channel AB, P11, P12, P15, P16, P17, and the characteristic points of the signals collected by the electrocardio channel CD, P21, P22, P25, P26, and P27, are matched one-to-one.

In some embodiments, the signals collected by the at least two sets of electrocardio channels may be matched based on a plurality of characteristic points, the plurality of characteristic points including a plurality of characteristic points of a plurality of types. For example, the R points, the Q points, and the S points are simultaneously used as the characteristic points to identify the R points, the Q points, and the S points of signals collected by each set of electrocardio channels to be matched with each other. Compared to matching based on one type of characteristic point, matching based on the plurality of characteristic points may improve the accuracy of the matching, especially in the case where there are many noise signals, and matching by the plurality of characteristic points may effectively exclude the interference of the noise signals to obtain an accurately restored electrocardio signal.

In 1330, a signal corresponding to matched characteristic points of the at least two sets of electrocardio channels are determined as the electrocardio signal.

In some embodiments, a signal corresponding to the same characteristic points (including one or more characteristic points) matched in the at least two sets of electrocardio channels are determined as the electrocardio signal. For example, referring to FIG. 12, the signal (e.g., the R wave corresponding to the R points) corresponding to matching the characteristic points (R points) P11, P12, P15, P16, P17 of the signals collected by the electrocardio channel AB with the characteristic points (R points) P21, P22, P25, P26, P27 of the signals collected by the electrocardio channel CD is determined to be the electrocardio signal. Similarly, the signal corresponding to the characteristic point Q may be the corresponding trough Q, the signal corresponding to the characteristic point S may be the corresponding trough S, etc. In some embodiments, the signal corresponding to matched plurality of characteristic points (including one or more characteristic points) in the at least two sets of electrocardio channels are determined as the electrocardio signal. In some embodiments, a characteristic point exceeding a deviation threshold may be screened and removed from the matched characteristic points of the at least two sets of electrocardio channels, and a signal corresponding to the screened and matched characteristic point may be determined as the electrocardio signal.

It should be noted that a plurality of different sets of electrocardio channels set at different positions in the human body have a certain difference in the electrocardio signal collected therefrom, as shown in FIG. 9. The difference described herein include a difference in time and a difference in strong signal strength of the characteristic points of different electrocardio channels. In some embodiments, matching the characteristic points of the collected signals of at least two sets of electrocardio channels may be screened by setting the deviation threshold. The deviation threshold may be a limit value of a positional deviation corresponding to the characteristic points of the at least two sets of electrocardio channels. In some embodiments, since the positional deviation corresponding to the characteristic points of the at least two sets of electrocardio channels exceeds the deviation threshold, the characteristic points involved do not form a match, and a signal component corresponding to the characteristic points is excluded as the noise signal from the electrocardio signal. In some embodiments, the deviation threshold may be expressed in a form of coordinates, e.g., the deviation threshold is set to (2.0 ms, 0.5 mV), i.e., the time threshold is 2.0 ms and a strength threshold is 0.5 mV. Because the electrocardio signals collected by the different electrocardio channels have a certain strength difference, in some embodiments, the strength difference of the characteristic points may be ignored. A limit value of the position deviation corresponding to the characteristic points of the at least two sets of electrocardio channels along a transverse coordinate is determined as the deviation threshold, i.e., the time threshold is determined as the deviation threshold. In some embodiments, the deviation threshold may be set to 2.0 ms. By setting the deviation threshold to filter the matched characteristic points, more interference from the noise signal may be excluded, thereby improving the accuracy of the obtained electrocardio signal. In some embodiments, in order to further improve the accuracy of the matched characteristic points, the characteristic points corresponding to the matched characteristic points need to appear in at least two sets of signals collected by the electrocardio channels. In some embodiments, when there are a plurality of sets of electrocardio channels, corresponding matched characteristic points need to appear in more than half of the signals collected by the electrocardio channels. It should be noted that the above regarding the time threshold or the strength threshold is not limited to the above mentioned range, and may be adjusted according to the actual situation.

In some embodiments, the signal corresponding to the same characteristic points (including one or more characteristic points) matched in the at least two sets of electrocardio channels may be determined as the electrocardio signal. For example, referring to FIG. 12, the signal (e.g., the R wave corresponding to the R point) corresponding to matching the characteristic points (R points) P11, P12, P15, P16, P17 of the signal collected by the electrocardio channel AB with the characteristic points (R points) P21, P22, P25, P26, P27 of the signal collected by the electrocardio channel CD is determined to be the electrocardio signal. Similarly, the signal corresponding to the characteristic point Q may be the corresponding trough Q, the signal corresponding to the characteristic point S may be the corresponding trough S, etc. In some embodiments, a signal corresponding to a plurality of matched characteristic points (e.g., any one or more of a peak of the R wave, a valley of an S valley, the R-S interval, or the like) in the at least two sets of electrocardio channels are determined as the electrocardio signal. In some embodiments, characteristic points exceeding the deviation threshold may be screened and removed from the matched characteristic points of the at least two sets of electrocardio channels, and the signal corresponding to the screened and matched characteristic points may be determined as the electrocardio signal.

It should be noted that the way of matching the characteristic points of the different electrocardio channels is not limited to the above-mentioned time threshold and/or the strength threshold, but also the way of matching the characteristic points of the different electrocardio channels using a differential slope or the combination of the time threshold, the strength threshold, and the differential slope to match the characteristic points of the different electrocardio channels.

In some embodiments, taking into account that different electrocardio channels are distributed in different positions, electrocardio waveforms collected are inherently different. In order to avoid processing such differences as the noise signal to the extent that the accuracy of the ultimately collected electrocardio signal is affected, it is possible to establish a conversion matrix of the electrocardio channel in different positions and convert signals collected by different electrocardio channels through the conversion matrix, which may eliminate different influences of different electrocardio waveforms collected in different positions.

In some embodiments, the signals collected by the different electrocardio channels may be converted according to a conversion matrix of the different electrocardio channels before extracting the characteristic points of the signals collected by each set of electrocardio channels. The conversion matrix refers to a conversion relationship between the electrocardio signal corresponding to the electrocardio channels at different positions and a reference electrocardio signal. The reference electrocardio signal may be an electrocardio signal at a position where a particular electrocardio channel is located, an electrocardio signal collected by another electrocardio acquisition device that may be designated as a reference, or a collected historical electrocardio signal of a user. After conversion by the conversion matrix, the electrocardio signals in the signals collected by the different electrocardio channels may have a relatively uniform or close representation. The conversion matrix may be used to reduce or eliminate a difference in electrocardio signals at different positions.

In some embodiments, a training set may be established to train an initial conversion matrix to obtain a trained conversion matrix, and the training set may include an electrocardio signal collected by the electrocardio channel at a position under low-noise interference and a reference electrocardio signal under low-noise interference. In some embodiments, the training set may be an electrocardio signal collected in a stationary state of the human body, as described in FIG. 8 and related descriptions. For example, in order to eliminate the effects of the distribution of muscles at different positions on the body, a reference electrocardio signal for training may be collected through the electrocardio channel (in which case at least two of the electrodes of the electrocardio channel may be distributed on the chest close to the heart) at the position of the heart, under the stationary state. It should be noted that conversion matrices between an electrocardio signal at each position on the human body and the reference electrocardio signal are different, and the conversion matrices are established separately for each position on the human body and are trained separately by a corresponding training set, to obtain the corresponding conversion matrix for each electrocardio signal collected at each position on the human body. The electrocardio signal collected at a certain position is input to the trained conversion matrix, and the trained conversion matrix outputs an electrocardio signal that is close to or consistent with the reference electrocardio signal. It is understandable that some of the parameters in the conversion matrix may be determined in the product development stage, and some of the parameters adapted to different human body morphologies may be left to be determined after the acquisition of the actual data of the user, which is conducive to reducing the difficulty of training after the user using the product, and to adapt to different users to improve the accuracy of the finally obtained electrocardio signal.

Before extracting the characteristic points of the signals collected by each set of electrocardio channels, the conversion of the signals collected by different electrocardio channels according to the conversion matrices of different electrocardio channels may reduce or eliminate the difference of the different electrocardio waveforms collected at different positions. Accordingly, the extraction of characteristic points may be performed based on the signal from the human body which are almost the same as the electrocardio signal, so that the extraction of characteristic points may be more accurate, and the purpose of obtaining the electrocardio signal with higher accuracy may be achieved in the end.

After extracting the characteristic points of the signals collected from each set of electrocardio channels, the characteristic points corresponding to different electrocardio channels are converted according to the conversion matrices between the different electrocardio channels, to make the matching of the characteristic points more accurate, and ultimately obtaining the electrocardio signal with a higher accuracy.

In some embodiments, the ultimately obtained electrocardio signal may be used to react in real-time to the user's heart rate variability (HRV) as well as heart rate, respiratory status information and other parameters to determine the physical state of the user, provide real-time feedback of the physical state to the user, and accompanied by the provision of referential health advice, exercise advice (e.g., switching of anaerobic exercise, aerobic duration of aerobic exercise), realizing the closed-loop process of health monitoring of the user. Additionally, the physical state of the user may include a tense state, a relaxed state, etc., and determining the physical state may be applied to a plurality of fields such as physical exercise, psychological testing, sleep monitoring, clinical health monitoring, etc.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

## Claims

1. An electrocardio monitoring system, comprising:
at least two sets of electrocardio channels, each of the at least two sets of electrocardio channels including at least two electrodes, wherein each of the at least two sets of electrocardio channels is configured to collect signals from a human body through the at least two electrodes, the signals including an electrocardio signal and a noise signal; and
a processing circuit, configured to extract the electrocardio signal from the signals collected by the at least two sets of electrocardio channels.

2. The electrocardio monitoring system of claim 1, wherein the at least two sets of electrocardio channels include a first electrocardio channel and a second electrocardio channel, the first electrocardio channel includes a first electrode and a second electrode, and the second electrocardio channel includes a third electrode and a fourth electrode.

3. The electrocardio monitoring system of claim 2, wherein the first electrode and the second electrode are located on two sides of a median sagittal plane of the human body, respectively, and the third electrode and the fourth electrode are located on the two sides of the median sagittal plane of the human body, respectively.

4. The electrocardio monitoring system of claim 1, wherein the at least two sets of electrocardio channels include a first electrocardio channel and a second electrocardio channel, the first electrocardio channel includes a first electrode and a second electrode, and the second electrocardio channel including the first electrode and a third electrode.

5. The electrocardio monitoring system of claim 1, wherein each set of the at least two sets of electrocardio channels includes two electrodes and a reference electrode, the reference electrode being located between the two electrodes.

6. The electrocardio monitoring system of claim 1, wherein a central distance between two electrodes corresponding to different electrocardio channels is not less than 5 mm.

7. **The** electrocardio monitoring system of claim 1, wherein the at least two electrodes corresponding to the at least two sets of electrocardio channels are made of metal fabric, and a thickness of the metal fabric is in a range of 10 µm to 5 mm.

8. **The** electrocardio monitoring system of claim 1, wherein the at least two sets of electrocardio channels are arranged on a same wearable body.

9. **The** electrocardio monitoring system of claim 8, wherein the wearable body includes a belt structure, the belt structure is configured to surround a waist region of the human body, and a size of the electrodes corresponding to the electrocardio channels along a first extending direction of the belt structure is greater than a size of the electrodes corresponding to the electrocardio channels along a second extending direction of the belt structure.

10. The electrocardio monitoring system of claim 1, wherein the at least two electrodes corresponding to one set of the at least two sets of electrocardio channels are respectively located at iliac bones on two sides of a median sagittal plane of the human body, and the at least two electrodes corresponding to another set of the at least two sets of electrocardio channels are respectively located at the lower back and on the two sides of the median sagittal plane of the human body.

11. The electrocardio monitoring system of claim 1, wherein the at least two sets of electrocardio channels are arranged on different wearable bodies.

12. The electrocardio monitoring system of claim 1, wherein the processing circuit includes a low-pass filter circuit, and a cutoff frequency of the low-pass filter circuit is not lower than 400 Hz.

13. The electrocardio monitoring system of claim 1, wherein the processing circuit includes a low-pass filter circuit, and a cutoff frequency of the low-pass filter circuit is not lower than 100 Hz.

14. The electrocardio monitoring system of claim 1, wherein a weight element is provided on the electrodes corresponding to one set of the at least two sets of electrocardio channels.

15. The electrocardio monitoring system of claim 14, wherein the weight element includes at least a portion of a circuit structure of the processing circuit.

16. The electrocardio monitoring system of claim 1, wherein the processing circuit is configured to extract the electrocardio signal from the signals collected by the at least two sets of electrocardio channels by:
extracting characteristic points of the signals collected by each set of the at least two sets of electrocardio channels, respectively;
matching the characteristic points of the signals collected by the at least two sets of electrocardio channels; and
determining a signal corresponding to matched characteristic points of the at least two sets of electrocardio channels as the electrocardio signal.

17. The electrocardio monitoring system of claim 16, wherein the matching the characteristic points of the signals collected by the at least two sets of electrocardio channels includes:
determining that the characteristic points of the signals collected by the at least two sets of electrocardio channels are successfully matched in response to that the characteristic points are within a deviation threshold range, wherein the deviation threshold includes a time threshold and/or an intensity threshold.

18. The electrocardio monitoring system of claim 16, wherein before extracting the characteristic points of the signals collected by each set of the at least two sets of electrocardio channels, the signals collected by different electrocardio channels are converted based on a transformation matrix between the different electrocardio channels.

19. The electrocardio monitoring system of claim 16, wherein after extracting the characteristic points of the signals collected by each set of the at least two sets of electrocardio channels, characteristic points corresponding to different electrocardio channels are converted based on a transformation matrix between the different electrocardio channels.

20. The electrocardio monitoring system of claim 18 or 19, wherein the transformation matrix is obtained based on historical electrocardio signals or electrocardio signals measured in real-time when the human body is in a stationary state.

21. An electrocardio monitoring method, comprising:
collecting signals from a human body through at least two sets of electrocardio channels, the signals including an electrocardio signal and a noise signal, each set of the at least two sets of electrocardio channels including at least two electrodes, wherein each of the at least two sets of electrocardio channels is configured to collect signals from the human body through the at least two electrodes; and
extracting the electrocardio signal from the signals collected by the at least two sets of electrocardio channels.

22. The method of claim 21, wherein the extracting the electrocardio signal from the signals collected by the at least two sets of electrocardio channels includes:
extracting characteristic points of the signals collected by each set of the at least two sets of electrocardio channels, respectively;
matching the characteristic points of the signals collected by the at least two sets of electrocardio channels; and
determining a signal corresponding to matched characteristic points of the at least two sets of electrocardio channels as the electrocardio signal.

23. The method of claim 21, wherein the at least two sets of electrocardio channels include a first electrocardio channel and a second electrocardio channel, the first electrocardio channel includes a first electrode and a second electrode, and the second electrocardio channel includes a third electrode and a fourth electrode.

24. The method of claim 23, wherein the first electrode and the second electrode are located on two sides of a median sagittal plane of the human body, respectively, and the third electrode and the fourth electrode are located on the two sides of the median sagittal plane of the human body, respectively.

25. The method of claim 21, wherein the at least two sets of electrocardio channels include a first electrocardio channel and a second electrocardio channel, the first electrocardio channel includes a first electrode and a second electrode, and the second electrocardio channel including the first electrode and a third electrode.

26. The method of claim 21, wherein a central distance between two electrodes corresponding to different electrocardio channels is not less than 5 mm.

27. The method of claim 21, wherein the at least two sets of electrocardio channels are arranged on a same wearable body.

28. The method of claim 21, wherein the at least two electrodes corresponding to one set of the at least two sets of electrocardio channels are respectively located at iliac bones on two sides of a median sagittal plane of the human body, and the at least two electrodes corresponding to another set of the at least two sets of electrocardio channels are respectively located at the lower back and on the two sides of the median sagittal plane of the human body.

29. The method of claim 21, wherein the at least two sets of electrocardio channels are arranged on different wearable bodies.

30. The method of claim 21, wherein extracting the electrocardio signal from the signals collected by the at least two sets of electrocardio channels is performed by a processing circuit, the processing circuit includes a low-pass filter circuit, and a cutoff frequency of the low-pass filter circuit is not lower than 400 Hz.

31. The method of claim 21, wherein extracting the electrocardio signal from the signals collected by the at least two sets of electrocardio channels is performed by a processing circuit, the processing circuit includes a low-pass filter circuit, and a cutoff frequency of the low-pass filter circuit is not lower than 100 Hz.

32. The method of claim 21, wherein a weight element is provided on the electrodes corresponding to one set of the at least two sets of electrocardio channels.
